# EUROPÄISCHE PATENTANMELDUNG

(11) **EP 3 178 809 A1**
(43) Veröffentlichungstag der Anmeldung: **14.06.2017**
(21) Anmeldenummer: 15199458.9
(22) Anmeldetag: 11.12.2015
(51) Int. Cl.: C07C 323/12, A61K 31/105, A61P 35/00

(54) **HYBRIDMOLEKÜL MIT STRUKTUR- UND WIRKUNGSELEMENTEN VON RESVERATROL UND DIALLYLSULFID**

(71) Anmelder: Albert-Ludwigs-Universität Freiburg, 79085 Freiburg (DE)
(72) Erfinder: Bauer, Georg, 79104 Freiburg (DE); Brückner, Reinhard, 79189 Bad Krozingen (DE); Braukmüller, Stefan, 79108 Freiburg (DE)
(74) Vertreter: Keller, Günter

(57) **Zusammenfassung**

Die vorliegende Erfindung betrifft Verbindungen der allgemeinen Formel (I) 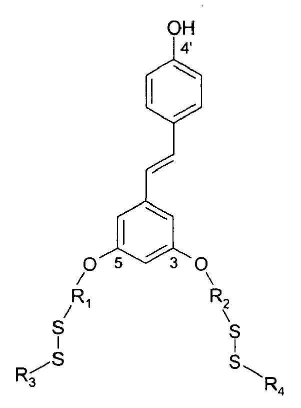 worin die Reste R₁ und R₂ unabhängig voneinander die Bedeutung (CH₂)ₙ haben, wobei n eine ganze Zahl zwischen 1 und 10 ist und R₃ und R₄ unabhängig voneinander ein Alkylrest mit 1 bis 10 C-Atomen, ein Alkenylrest mit 2 bis 10 C-Atomen, ein Allylrest oder ein Cycloalkylrest mit 5 bis 10 C-Atomen ist, sowie deren Verwendung als Arzneimittel, die insbesondere der Tumortherapie dienen.

## Beschreibung

In den letzten Jahrzehnten wurden die Hintergründe des Tumorzellphänotyps, der durch die Kombination von membranständiger NADPH-Oxidase (NOX1), membranständiger Katalase sowie membranständiger Superoxiddismutase gekennzeichnet ist, aufgeklärt (Bauer G, et al., Chimia 62: 1-9 (2008); Heinzelmann et al., Biol. Chem. 391, 675-693 (2010); Bauer G, Anticancer Res. 32: 2599-2624 (2012); Bauer G, Anticancer Res. 34: 1467-1482 (2014); Scheit K et al., Carcinogenesis 36: 400-411 (2015); Bauer G., Redox Biol 6: 353-371 (2015)).

Nach Hemmung oder Inaktivierung der membranständigen Katalase der Tumorzellen werden zwangsläufig aufgrund der konstitutiv aktivierten NOX1 mehrere biochemisch detailliert charakterisierte interzelluläre Signalwege reaktiviert, die zum selektiven Zelltod der Tumorzellen durch Apoptose führen. Auf der Grundlage dieses Prinzips wird ein neuer Weg einer selektiven Tumortherapie vorgeschlagen.

Ein vielversprechender Weg der Inaktivierung der protektiven Katalase dürfte die Bildung von Singulettsauerstoff sein, der nach Reaktion mit Histidin im aktiven Zentrum der Katalase zu deren Inaktivierung führt. Es wird vorgeschlagen, dass die besondere Redoxsituation von Tumorzellen dazu geeignet ist, die Bildung von extrazellulärem Singulettsauerstoff durch die Tumorzellen selbst anzuregen. Ein wesentlicher Schlüssel für die zelleigene Singulettsauerstoffbildung ist die Modulation der verfügbaren Stickstoffmonoxid- (NO) Konzentration in der Tumorzelle. Es wird davon ausgegangen, dass die Modulatoren der NO-Konzentration synergistisch mit Stimulatoren der NADPH-Oxidase bei der Singulettsauerstoffbildung und Katalaseinaktivierung zusammenarbeiten.

Hybride bifunktionale Antitumor-Mittel wurden beispielsweise von Musso et al., Biochemical Pharmacology (2015), 96, S 297-305 beschrieben, jedoch handelt es sich hierbei vor allem um hoch zytotoxische Substanzen wie DNA modifizierende Verbindungen.

Erfindungsgemäß werden Schlüsselstrukturelemente von zwei synergistisch wirkenden Komponenten, nämlich dem NO-Dioxygenase (NOD)-Hemmstoff Diallyldisulfid (DADS) und dem NOX1-Stimulator Resveratrol in Hybridmolekülen zusammengeführt.

Ein Vorteil von Hybridmolekülen gegenüber der gleichzeitigen Gabe der Einzelsubstanzen liegt darin, dass im Fall des Hybridmoleküls Aufnahme, Bioverfügbarkeit, Ausscheidung, Erreichen des Zielortes, Erreichen bestimmter Wirkkonzentrationen für beide Komponenten stets gleich sind, im Gegensatz zur Gabe der Einzelsubstanzen. Dies ermöglicht eine besser zu steuernde Wirkung. Außerdem ist eine Testung in vivo aufgrund der geringeren Komplexität der Testansätze praktikabler und leichter durchführbar. Statt Schachbrett-artige Kombinationen der Konzentrationen der Einzelsubstanzen zu untersuchen, ergibt sich für die Testung des Hybridmoleküls eine einfache, lineare Dosis-Wirkungsbeziehung.

Es wurden Verbindungen aus einem Strukturraum von Hybridmolekülen, die Schlüsselstruktur- und Wirkungselemente des NO-Dioxygenase (NOD)-Hemmstoffs Diallyldisulfid und des NADPH Oxidase (NOX1)-Stimulators Resveratrol miteinander verbinden, synthetisiert. Dabei wurde sichergestellt, dass die beiden Einzelwirkungen (Hemmung von NOD und Stimulation von NOX1) erhalten blieben.

Die Hybridmoleküle regen durch das Zusammenspiel der beiden Teilfunktionen in optimaler Weise Tumorzellen zur Bildung von extrazellulärem Singulettsauerstoff an, der die protektive, membranständige Katalase der Tumorzellen inaktiviert und dadurch die Apoptose der Zellen über interzelluläre, ROS-abhängige Apoptose-auslösende Signalwege ermöglicht. Es wird angenommen, dass die erfindungsgemäßen Hybridmoleküle vor allem auf Zielstrukturen in der Zellmembran von Tumorzellen wirken und aufgrund des angenommenen Wirkmechanismus hochspezifisch die Apoptose von Tumorzellen hervorrufen, aber kaum unerwünschte Nebenwirkungen gegenüber Nicht-Tumor-Zellen aufweisen.

Die biochemische und biologische Wirkung der Hybridmoleküle hängt von drei biochemischen Charakteristika ab, die spezifisch für Tumorzellen sind. Dies sind
1) extrazelluläre Superoxidanionproduktion durch membranständige NOX1,
2) membranständige Katalase und
3) die Fähigkeit, nach Hemmung der NOD extrazellulär Singulettsauerstoff zu produzieren. Durch die erfindungsgemäßen Hybridmoleküle wird, wie durch entsprechende in-vitro-Versuche nachgewiesen, Apoptose selektiv in Tumorzellen ausgelöst. Daher ist auch ein hohes Maß an Selektivität und Wirksamkeit für Tumorzellen in vivo zu erwarten.

Die Hybridmoleküle bündeln in sich die Synergiewirkung, die auch für die Kombination der Einzelstoffe Diallyldisulfid und Resveratrol nachgewiesen werden kann. Durch diese Bündelung des Synergieeffektes in einem Hybridmolekül ergeben sich erhebliche Vereinfachungen, sowie praktische Vorteile bei der Testung der biologisch und therapeutisch wichtigen Parameter und der Therapie. Statt Schachbrett-artiger Kombinationen von Konzentrationen der Einzelsubstanzen kann nun mit einer einfachen linearen Dosiswirkungsbeziehung eines Hybridmoleküls gearbeitet werden. Zudem muss nun lediglich die Pharmakologie einer Substanz erarbeitet werden und es ist sichergestellt, dass sich die beiden Wirkgruppen, die für die beabsichtigte Synergiewirkung notwendig sind, auch gleichzeitig und in gleicher Konzentration die malignen Zielzellen erreichen. Als weiterer Vorteil wird angesehen, dass die beiden Wirkgruppen gemeinsam an den Zielort (die Zellmembran von Tumorzellen) gebracht werden und dort an den Zielstrukturen ihre Wirkung entfalten können.

Die Austestung der Wirksamkeit der Hybridmoleküle und deren weitere Charakterisierung in vitro zeigten, dass die Hybridmoleküle bis zu 1000-fach wirksamer als der Ausgangsstoff Diallyldisulfid und 100-fach wirksamer als das Referenztumortherapeutikum Taxol sind. Die Wirksamkeit der Hybridmoleküle konnte durch die Zugabe der Einzelkomponenten nicht weiter gesteigert werden. Auch ein erstmalig beobachteter synergistischer Effekt aus der Kombination von Taxol mit Resveratrol konnte die Wirksamkeit des Hybridmoleküls aus Diallyldisulfid und Resveratrol nicht übertreffen.

Damit eröffnet sich ein rationaler und wirtschaftlicher Weg zur Herstellung potenziell hochwirksamer und hochselektiver Tumormedikamente, von denen aufgrund der niedrigen Wirkkonzentrationen ein Minimum an Nebenwirkungen erwartet werden kann. Die Bündelung des Synergieeffekts in einem Molekül senkt zudem die Entwicklungskosten des potenziellen Medikamentes erheblich. Darüber hinaus eröffnet die Entwicklungsarbeit zur Synthese des Hybridmoleküls die Möglichkeit, die therapeutische Wirksamkeit und andere relevante Parameter weiter zu optimieren, indem weitere Hybridmoleküle aus dem definierten Strukturraum hergestellt werden.

Die vorliegende Erfindung wird näher erläutert durch die Figuren und die Beispiele. Bevorzugte Ausführungsformen der vorliegenden Erfindung sind Gegenstand der Ansprüche. Im Rahmen der vorliegenden Erfindung werden verschiedene Abkürzungen verwendet, die in der nachfolgenden Tabelle 1 zusammengefasst sind:

**Tabelle 1: Abkürzungen und Erläuterungen**

| | |
|---|---|
| 3-AT | 3-Aminotriazol (Katalaseinhibitor) |
| ABH | 4-Aminobenzoylhydrazid (Peroxidase-Inhibitor) |
| AEBSF | 4-(2-Aminoethyl)benzenesulfonyl fluoride (NOX-Inhibitor) |
| Casp8 | Caspase8 |
| CAT | Katalase |
| CATFe^{III} | Katalase (natives Enzym) |
| CATFe^{IV}=O⁺ | Compound I der Katalase (aktives Intermediat) |
| CATFe^{IV}=O | Compound II der Katalase (inaktives Intermediat) |
| CATFe^{III}O₂⁻ | Compound III der Katalase (inaktives Intermediat) |
| CATFe^{III}NO | Katalase/NO complex |
| Cpd I, II, III | Compound I, II, III |
| DUOX | Duale Oxidase |
| EUK-134 | [chloro([2,2'-[1,2-ethanediylbis[(nitrilo-κN)methylidyne]]bis[6-methoxyphenolato-κO]]]-manganese (Katalasemimetikum) |
| FeTPPS | 5-, 10-, 15-, 20-Tetrakis(4-sulfonatophenyl)porphyrinato iron(III) chloride (Peroxynitrit-Fänger) |
| HIS | Histidin (Singulett-Sauerstoff-Sscavenger) |
| L-NAME | N-omega-nitro-L-arginine methylester hydrochloride (NO-Synthase (NOS)-Inhibitor) |
| LPO | Lipid-Peroxidation |
| MANN | Mannitol (Hydroxylradikal-Fänger) |
| NO | Nitric oxide (Stickstoffmonoxid) |
| NOD | NO-Dioxygenase |
| NOS | NO-Synthase |
| NOX | NADPH-Oxidase |
| POD | Peroxidase |
| PODFe^{III} | Peroxidase (Natives Enzym) |
| PODFe^{IV}=O·⁺ | Compound I der Peroxidase |
| PODFe^{IV}=O | Compound II der Peroxidase |
| PON | Peroxynitrit |
| ROS | Reactive oxygen species (Reaktive Sauerstoffspezies) |
| SA | Salicylic acid (Salicylsäure) |
| siRNA | small interfering RNA |
| SOD | Superoxiddismutase |
| TAU | Taurin (HOCI-Fänger) |
| TGF-beta1 | Transforming growth factor-beta1 |
| O₂^{.-} | Superoxid- Anion |
| H₂O₂ | Hydrogen peroxide (Wasserstoffperoxid) |
| HO₂⁻ | Anion des Wasserstoffperoxids |
| HO·₂ | Hydroperoxyl-Radikal |
| ·OH | Hydroxylradikal |
| OH⁻ | Hydroxylanion |
| ¹O₂ | Singulettsauerstoff |
| HOCI | Hypochlorige Säure |
| ·NO₂ | Nitrogen-dioxide (Stickstoffdioxid) |
| NO₂⁻ | Nitrit |
| NO₃⁻ | Nitrat |
| ONOO⁻ | Peroxynitrit |
| ONOOH | Peroxynitritsäure |
| ONOO· | Peroxynitritradikal |
| NO⁺ | Nitrosoniumion |
| N₂O₃ | Dinitrogentrioxid |
| N₂O₄ | Dinitrogentretraoxide |
| ONOONO | Nitrosoperoxy-nitric oxide; isomer of N₂O₄ |
| ONOOCOO⁻ | Nitrosoperoxycarboxylat |
| CO₃·⁻ | Carbonatradikal |
| CO₃²⁻ | Carbonat |

Diese Abkürzungen werden u.a. in den Figuren verwendet.

Gegenstand der vorliegenden Erfindung sind Verbindungen der allgemeinen Formel (I) 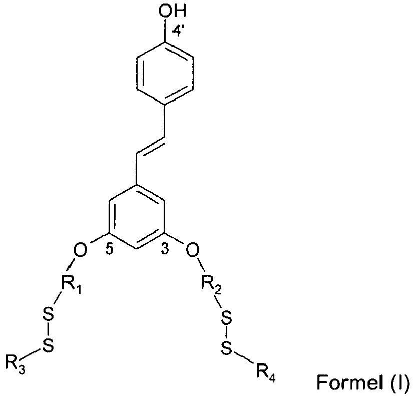 worin die Reste R₁ und R₂ unabhängig voneinander die Bedeutung (CH₂)ₙ haben, wobei n eine ganze Zahl zwischen 1 und 10 ist und R₃ und R₄ unabhängig voneinander ein Alkylrest mit 1 bis 10 C-Atomen, ein Alkenylrest mit 2 bis 10 C-Atomen, ein Allylrest oder ein Cycloalkylrest mit 5 bis 10 C-Atomen ist.

Besonders bevorzugt ist die Verbindung der nachfolgenden Formel (II):

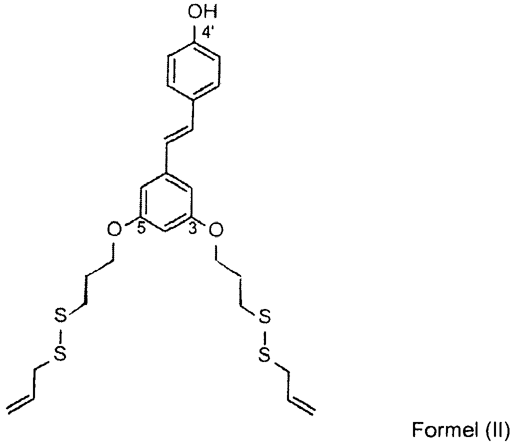

In einer weiteren Ausführungsform betrifft die Erfindung Arzneimittel, die eine pharmakologisch ausreichende Menge einer Verbindung der Formel (I) oder der Formel (II) oder deren pharmazeutisch annehmbaren Salze beinhalten.

Bei den pharmazeutisch annehmbaren Salzen der erfindungsgemäßen Verbindungen handelt es sich um solche, die einerseits gut herstellbar und andererseits bei Verabreichung am Säugerorganismus gut verträglich sind. In bevorzugter Ausführungsform handelt es sich hierbei um Natriumsalze, Kaliumsalze oder andere dem Fachmann wohlbekannte pharmazeutisch unbedenkliche Salze.

Die erfindungsgemäßen Arzneimittel sind besonders geeignet zur Behandlung von Tumorerkrankungen. Ganz besonders geeignet sind die Verbindungen zur Behandlung von verschiedenen Karzinomerkrankungen wie Lungenkarzinom, Magenkarzinom, Mammakarzinom oder auch zur Behandlung von Tumorerkrankungen des lymphatischen Systems wie Lymphdrüsenkrebs.

In bevorzugter Weise werden die erfindungsgemäßen Verbindungen bei der Behandlung einer Tumorerkrankung eingesetzt, wobei die Verabreichung der Wirkstoffe peroral, also in Form von Tabletten, Kapseln, Pellets oder Sirup erfolgen kann.

Besonders bevorzugt ist auch die parenterale Verabreichung, also in Form von Injektionslösungen, Infusionslösungen oder auch in Form von Retardformulierungen. Die erfindungsgemäßen Verbindungen können dabei in Form von den Wirkstoff enthaltenden Matrixstrukturen, die beispielsweise aus Polylactiden bestehen, verabreicht werden. Diese intraperitoneal verabreichbaren Arzneimittel können beispielsweise subkutan, insbesondere bei der Behandlung von lokal identifizierbaren Tumoren, in dessen Nähe verabreicht werden und lösen sich im Lauf der Zeit unter kontinuierlicher Freigabe des Wirkstoffs auf. Dadurch ist eine Therapie über einen definierten Zeitraum mit kontinuierlicher Freigabe des Wirkstoffes möglich, ohne dass der Wirkstoff immer wieder gespritzt werden muss, was zur Beeinträchtigung des zu behandelnden Patienten führen kann.

Die vorliegende Erfindung und der zugehörige biochemische Hintergrund werden näher anhand der Figuren erläutert, wobei die biochemischen Grundlagen durch Versuche, häufig mit Zellkulturen erarbeitet wurden. Diese Versuchsergebnisse wurden in den Figuren graphisch dargestellt und mit der zugehörigen Erläuterung verdeutlicht.

Ohne an eine Theorie gebunden sein zu wollen, wird davon ausgegangen, dass die erfindungsgemäßen Hybridmoleküle aufgrund der nachfolgend skizzierten Mechanismen funktionieren:

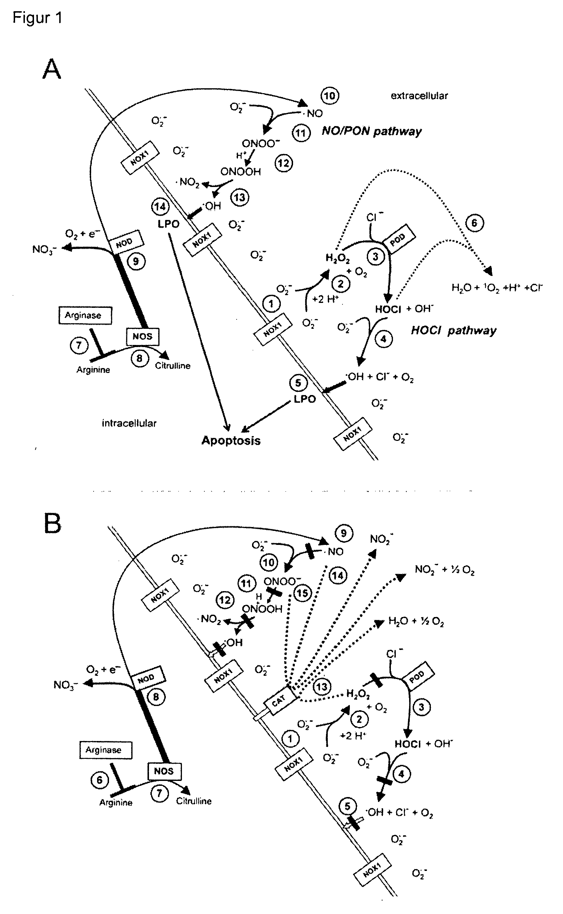

**Figur 1A** **und B:** *Prinzip des interzellulären Apoptose-auslösenden HOCI- und NOlPeroxynitritsignaling maligner Zellen und der protektiven Wirkung membranständiger Katalase von Tumorzellen gegen diese Signalwirkung.*

Interzelluläre ROS-abhängige Signalwege (HOCI- und NO/Peroxynitritsignalweg) zur Induktion der Apoptose in malignen Zellen

Transformierte Zellen sind durch die Expression einer membranständigen NADPH-Oxidase (NOX1) charakterisiert, die extrazelluläre Superoxidanionen generiert **(****Figur 1** **A,** Reaktion (1). Durch Dismutation der Superoxidanionen entstehendes H₂O₂ (2) wird von einer DUOX-kodierten Peroxidase zu HOCI umgesetzt (3). HOCl reagiert mit Superoxidanionen unter Bildung von ·OH-Radikalen (4), die Lipidperoxidation (LPO, (5)) und dadurch Apoptose auslösen. Bei Vorliegen eines Überschusses von H₂O₂ kommt es zu einer Konsumreaktion von HOCI, die zur Abschwächung des HOCl-Signalweges führt (6). Die intrazelluläre Konzentration an Arginin wird durch Arginase kontrolliert (7). Arginin dient der NO-Synthase (NOS) als Substrat zur Herstellung des Radikals Stickstoffmonoxid (NO) (8). ·NO-Dioxygenase (NOD) reguliert die freie Konzentration an ·NO (9), indem sie einen Teil der ·NO-Konzentration in Nitrat umsetzt. ·NO passiert die Zellmembran (10) und reagiert mit Superoxidanionen unter Bildung von Peroxynitrit (ONOO⁻) (11). Protonen, die von membranständigen Protonenpumpen generiert werden führen zur Bildung von Peroxynitritsäure (ONOOH) aus Peroxynitrit (12). ONOOH zerfällt in ·NO₂ und ·OH- Radikale (13), wobei letztere die Lipidperoxidation (14) und Apoptose auslösen.

**Figur 1** **B** zeigt, dass Tumorzellen sowohl den HOCI-Weg (1)-(5) als auch den ·NO/Peroxynitritweg (7)-(12) durch membranständige Katalase hemmen. Dies erfolgt durch Abbau von H₂O₂ (13), Oxidation von NO (14) und Zerstörung von Peroxynitrit (15).

**Figur 2****:** *Biochemische Mechanismen der protektiven Wirkung membranständiger Katalase von Tumorzellen.*

Membranständige Katalase von Tumorzellen hemmt den NO/Peroxynitrit- und den HOCl-Signalweg: zugrundeliegende biochemische Mechanismen.

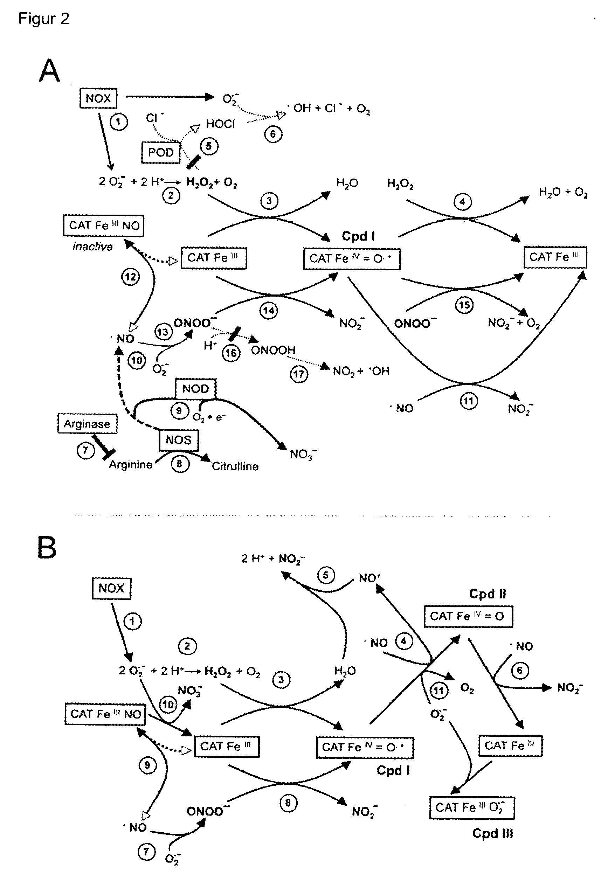

**Figur 2** **A** zeigt die membranständige NADPH Oxidase (NOX1) (1), die extrazelluläre Superoxidanionen (O₂·⁻) generiert. Diese dismutieren zu H₂O₂ (#2), welches durch Katalase (CATFe^{III}) unter Bildung von Compound I (CATFe^{IV}=O·⁺) zersetzt wird (3. 4). Dadurch wird der HOCI-Weg (5, 6) wirkungsvoll gehemmt.

Arginase (7) kontrolliert die verfügbare Konzentration an Arginin, welches von ·NO Synthase (NOS) als Substrat zur Synthese von NO verwendet wird (8). Die Konzentration von ·NO wird durch die Aktivität von NO Dioxygenase (NOD) (9) gemindert. Die verfügbare Konzentration NO (10) kann nun entweder durch Compound I der Katalase oxidiert werden (11), bei entsprechend hoher Konzentration reversibel die Katalase hemmen (12) oder mit Superoxidanionen zu Peroxynitrit reagieren (13). Peroxynitrit wird wiederum durch Katalase in einem Zweistufenmechanismus abgebaut (14), (15).

Die vielseitigen Reaktionsmöglichkeiten zwischen NO und Superoxidanionen mit Katalase sind für die im Rahmen dieser Erfindung wichtigen Kontrollvorgänge von besonderer Bedeutung und sollen daher in der nachfolgenden **Figur 2** **B** detailliert betrachtet werden. Die durch NOX1 generierten Superoxidanionen (1) können zu H₂O₂ dismutieren (2), welches mit Katalase (CATFe^{III}) zu Compound I (Cpd I) (CATFe^{IV}=O·⁺) reagiert (3). Compound I kann nun NO zum Nitrosoniumion (NO⁺) oxidieren (4), welches mit Wasser zu NO₂⁻ reagiert (5). Das entstehende inaktive Intermediat der Katalase Compound II (CATFe^{IV}=O) reagiert nun mit einem zweiten Molekül NO (6), wobei NO₂⁻ und native Katalase entstehen. NO kann alternativ mit Superoxidanionen zu Peroxynitrit (ONOO⁻) reagieren (7), welches ebenfalls mit Katalase unter Bildung von Compound I reagieren kann (8). Dem Abbau von aus NO entstandenem Peroxynitrit und der Oxidation von NO durch Katalase steht das Potenzial von Katalase gegenüber, Katalase reversibel zu hemmen (9). Voraussetzung dafür ist allerdings Konzentration an NO im mikromolaren Konzentrationsbereich. Der inaktive CATFe^{III}NO-Komplex kann durch freie Superoxidanionen wieder reaktiviert werden, wobei Nitrationen entstehen (10). Dieser Superoxidanionen-vermittelten Aufhebung der NO-abhängigen Hemmung der Katalase steht die direkte hemmende Wirkung von Superoxidanionen entgegen, die durch die Reduktion von Compound I zum inaktiven Compound II oder durch die Bildung von Compound III (CATFe^{III}O₂·⁻) bewirkt wird (11).

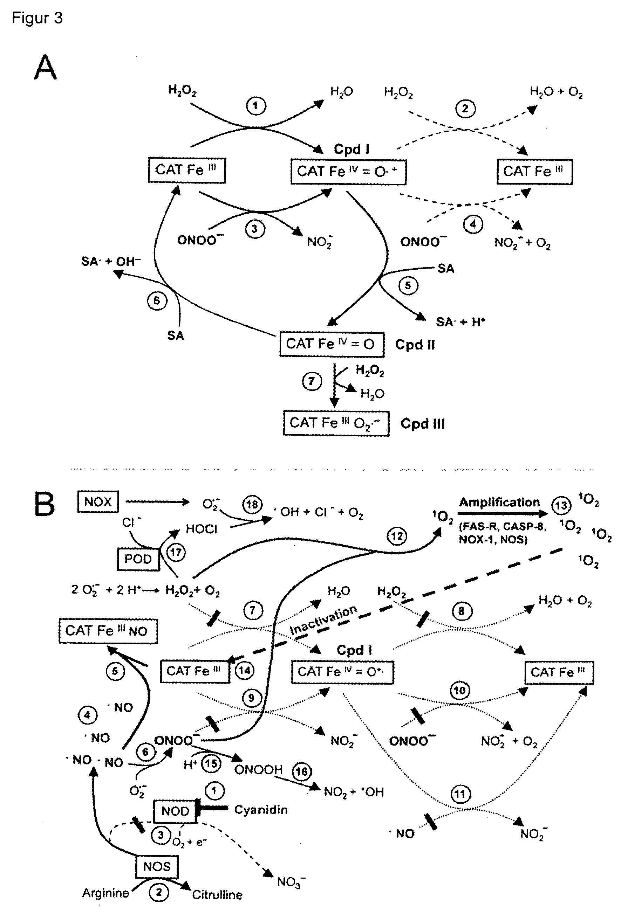

**Figur 3****:** *Direkte Hemmung von Katalase (A) und Singulettsauerstoff vermittelte Inaktivierung von Katalase: zwei Wirkmechanismen tumorpräventiv oder therapeutisch wirksamer sekundärer Pflanzenstoffe.*

Hemmung und Inaktivierung von Katalase durch sekundäre Pflanzenstoffe

Interessanterweise führen bestimmte sekundäre Pflanzenstoffe mit tumorpräventiver oder (potenzieller) therapeutischer Antitumorwirkung zur Hemmung oder Inaktivierung der membranständigen protektiven Katalase von Tumorzellen (Scheit K et al., Carcinogenesis 36: 400-411 (2015)). Dabei können zwei grundlegend verschiedene Mechanismen unterschieden werden, nämliche die direkte Hemmung der Katalase z. B. durch Salicylsäure und die Modulation der Bildung von Singulettsauerstoff, der Katalase inaktiviert, z. B. durch die Wirkung von z.B. Anthocyanidinen (Scheit K et al., Carcinogenesis 36: 400-411 (2015)).

Salicylsäure und ihre Derivate hemmen Katalase direkt, wie **Figur 3** **A** zeigt. Dabei wird die entweder aus der Reaktion von Katalase mit H₂O₂ (1) oder Peroxynitrit (3) entstehende Zwischenstufe Compound I (Cpdl) (CATFe^{IV}=O·⁺) durch Salicylsäure (SA) zum inaktiven Compound II (CATFe^{IV}=O) reduziert (5). Ein zweites Molekül Salicylsäure kann nun Compound II wieder in native Katalase überführen (6). Alternativ kann Compound II durch H₂O₂ zum ebenfalls inaktiven Compound III (CATFe^{III}O₂·⁻) überführt werden (7). Dieser peroxidative Zyklus der Katalase in Gegenwart von Salicylsäure hemmt somit den eigentlichen Katalasezyklus des Enzyms.

Eine Vielzahl von untersuchten sekundären Pflanzenstoffen führt hingegen zu einer Modulation des NO-Stoffwechsels der Tumorzellen, was mittelbar die Inaktivierung der Katalase durch Singulettsauerstoff bewirkt **(****Figur 3 B)****.** Hier werden die Mechanismen beschrieben, die vor kurzem von uns für Cyanidin nachgewiesen wurden (Scheit K et al., Carcinogenesis 36: 400-411 (2015)). Cyanidin hemmt die ·NO Dioxygenase (NOD) (1), wodurch der Verbrauch von NOS generiertem ·NO (2) durch NOD (3) gemindert wird und ein starker Anstieg von freiem NO erzielt wird (4). Dieser ist ausreichend für eine lokale Hemmung einzelner Katalasemoleküle auf der Zellmembran (5). Als unmittelbare Konsequenz dieser Hemmung werden nun Peroxynitrit (6), (9), (10) und H₂O₂ (7), (8) nicht mehr durch die gehemmten Katalasemoleküle abgebaut und die Oxidation von ·NO durch Katalase wird vermindert (11). Freies Peroxynitrit und H₂O₂ können nun zu Singulettsauerstoff reagieren (12), der zunächst durch Liganden-unabhängige Stimulierung des FAS-Rezeptors zur Aktivierung von Caspase-8 führt. Diese stimuliert die Aktivität von NOX und induziert unter Umständen NOS, so dass es zu einer Autoamplifikation der Bildung von Singulettsauerstoff (13) und nachfolgender Inaktivierung weiterer Katalasemoleküle kommt (14). Daraufhin können nun der ·NO/Peroxynitritweg (15), (16) und der HOCI-Weg (17), (18) ungebremst Apoptose in der Tumorzelle auslösen.

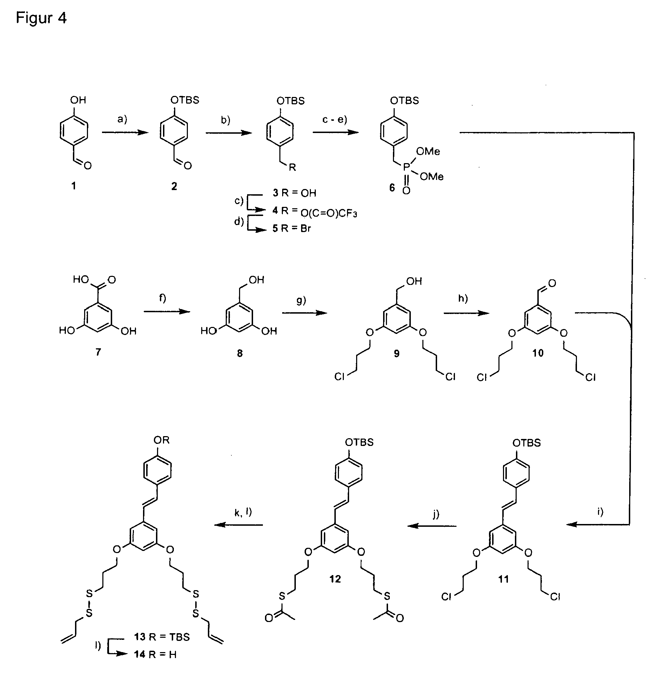

**Figur 4****:** *Synthese eines bevorzugten Hybridmoleküls mit Struktur- und Wirkungselementen von Diallyldisulfid und Resveratrol.* Der Syntheseweg ist detailliert in den Beispielen beschrieben.

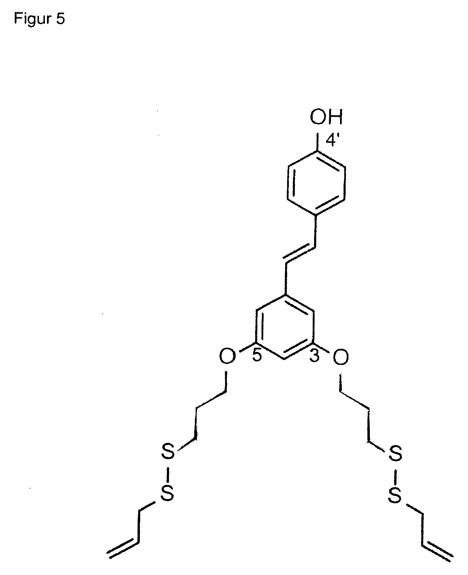

**Figur 5****:** *Struktur des besonders bevorzugten Hybridmoleküls*

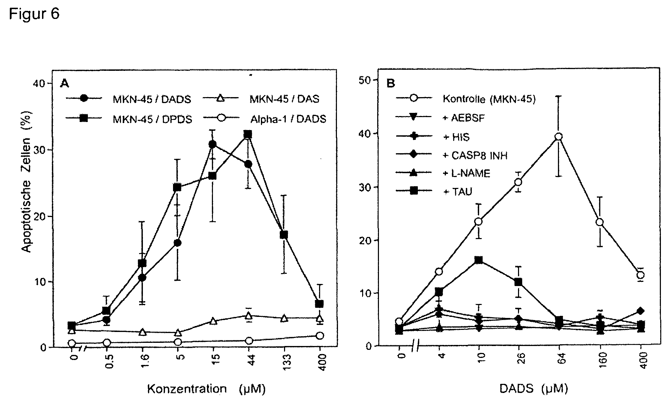

**Figur 6****:** *Diallyldisulfid und Dipropyldisulfid, nicht aber Diallylsulfid lösen selektiv in Tumorzellen interzelluläres Signaling und Apoptose aus.*
**A.** 12 500 humane Magenkarzinomzellen MKN-45 oder 10 000 nichtmaligne, diploide humane Fibroblasten Alpha-1 wurden in Doppelansätzen in Serum-haltigen Medium mit den angegebenen Konzentrationen an Diallyldisulfid (DADS), Dipropyldisulfid (DPDS) oder Diallylsulfid (DAS) versetzt. Nach 3 Stunden bei 37 ° C, 5 % CO₂ wurden die Prozentsätze apoptotischer Zellen bestimmt. DADS und DPDS führen bei den Tumorzellen, nicht aber bei den normalen Zellen konzentrationsabhängig zur Apoptose. Das Monosulfid DAS zeigt keine Aktivität.
**B.** Vor der Zugabe von DADS zu MKN-45 unter Standardbedingungen (A) erhielten die Ansätze keine weiteren Zusätze (Kontrolle) oder 100 µM des NOX-Inhibitors AEBSF, 2 mM des Singulettsauerstofffängers Histidin (HIS), 25 µM Caspase-8-Inhibitor, 2.4 mM des NOS-Inhibitors L-NAME oder 50 mM des HOCI-Fängers Taurin. Nach 3 Stunden bei 37 ° C, 5 % CO₂ wurden die Prozentsätze apoptotischer Zellen bestimmt. Das Ergebnis zeigt, dass die durch DADS ausgelöste Apoptose von der Wirkung von Singulettsauerstoff, Superoxidanionen, Caspase-8, NO und HOCI abhängig ist.

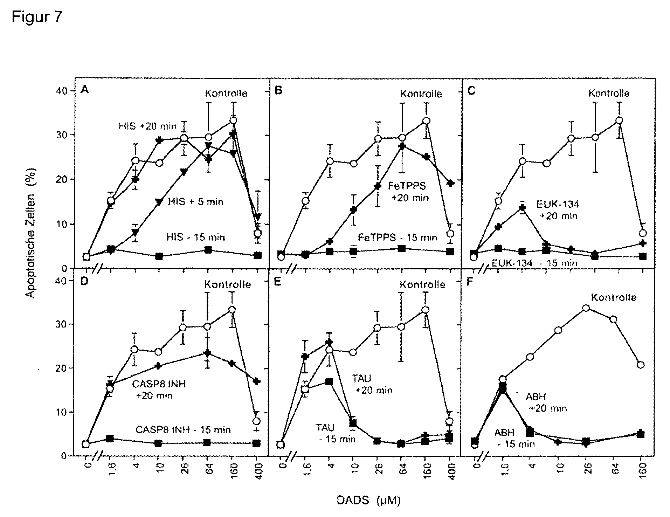

**Figur 7****:** *Diallyldisulfid induziert einen Zweistufenmechanismus*

MKN-45 Tumorzellen (Standardbedingungen) erhielten die angegebenen Inhibitoren entweder 15 Minuten vor DADS ("-15 min") oder 20 Minuten nach DADS-Zugabe ("+ 20 min"). Kontrollen blieben frei von Inhibitoren, erhielten aber DADS. Nach 3 Stunden bei 37°C, 5 % CO₂ wurden die Prozentsätze apoptotischer Zellen in den Doppelansätzen bestimmt. Inhibitoren: 2 mM des Singulettsauerstofffängers Histidin (A), 25 µM des Peroxynitritfängers FeTPPS (B), 0.5 µM des Katalasemimetikums EUK-134 (C), 25 µM Caspase-8-Inhibitor (D), 50 mM des HOCl-Fängers Taurin (E), 150 µM des Peroxidasehemmers 4-Aminobenzoylhydrazide (ABH) (F). Das Ergebnis erlaubt die Differenzierung zwischen einem frühen, Singulettsauerstoff-abhängigen Schritt und nachfolgendem Interzellulären Signaling über den NO/Peroxynitrit und den HOCI-Signalweg. Für die Bildung von Singulettsauerstoff im frühen Schritt werden Superoxidanionen, H₂O₂, ·NO und Peroxynitrit benötigt.

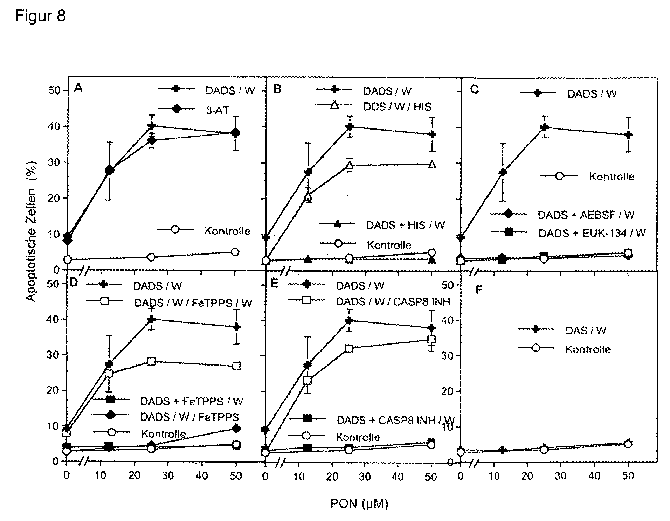

**Figur 8****:** *DADS führt zur Inaktivierung der protektiven Katalase von Tumorzellen*
**A**. Humane MKN-45 Tumorzellen enthielten entweder zunächst keine Zusätze (Kontrolle), 50 mM des Katalasehemmers 3-AT oder wurden für 20 Minuten mit 150 µM DADS behandelt. Nach einem Waschschritt und der Verdünnung der Zellkonzentration auf 6000 Zellen / 100 µl Ansatz wurde Peroxynitrit (PON) in den angegebenen Konzentrationen zugegeben. Nach 100 Minuten erfolgte die Bestimmung der apoptotischen Zellen in Doppelansätzen. Wie die Kontrolle zeigt, sind die Tumorzellen vor der Apoptose-auslösenden Wirkung von Peroxynitrit geschützt. Nach Hemmung der Katalase mittels 3-AT wird der Schutz aufgehoben und Apoptose induziert. DADS-Vorbehandlung erzielt den gleichen Effekt wie der Katalasehemmstoff, allerdings über einen komplexeren Mechanismus, wie B-E zeigen. DAS zeigt keine Wirkung auf die protektive Katalase (F).
B-E: Die angegebenen Inhibitoren (50 mM Histidin (B), 100 µM AEBSF, 2 µM EUK-134 (C), 25 µM FeTPPS (D) oder 25 µM Caspase-8-Inhibitor (E) wurden entweder vor DADS (150 µM) zugegeben ("DADS + x") oder nach erfolgter DADS-Behandlung und dem Waschschritt ("DADS/W/x"). Anschließend folgte die Zugabe der angegebenen Konzentrationen von Peroxynitrit (PON) und die Bestimmung der apoptotischen Zellen nach 100 min. Das Ergebnis zeigt, dass die Inaktivierung der Katalase durch DADS über die Bildung von Singulettsauerstoff erreicht wird. Singulettsauerstoff entsteht dabei aus zelleigenem H₂O₂ und Peroxynitrit.

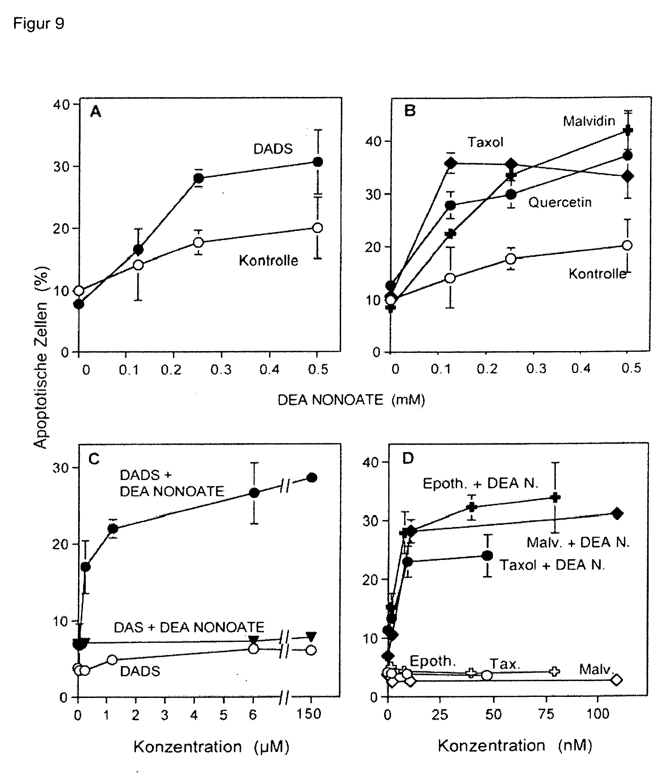

**Figur 9****:** *DADS, Anthocyanidine undTaxol hemmen die NO Dioxygenase*

Unter Verwendung des bei Scheit und Bauer, 2015 vorgestellten Tests auf Hemmung der ·NO Dioxygenase wird gezeigt, dass Taxol und DADS zur Hemmung dieses Enzyms führen, ebenso wie Malvidin, Quercitin und Epothilon B. Das Prinzip des Tests beruht darauf, dass durch Hemmung der NOD die Wirkung des exogenen NO Donors DEA NONOate auf Superoxidanionen-produzierende Zellen gesteigert wird. Die Messung erfordert, dass durch geeignete Maßnahmen, wie bei Scheit und Bauer, 2015 beschrieben, die Tumorzellkatalase maximal inhibiert, die Hemmwirkung von H₂O₂ auf ·NO und Peroxynitrit durch Anwesenheit eines Katalasemimetikums verhindert und de novo Synthese von ·NO durch die Anwesenheit des NOS Inhibitors L-NAME verhindert wird.

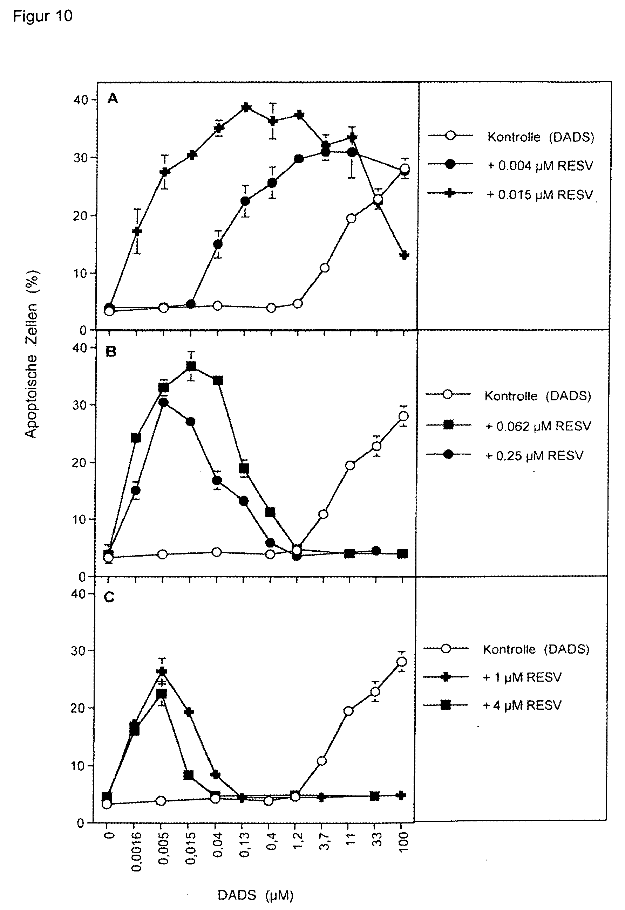

**Figur 10****:** *Synergistischer Effekt zwischen DADS und Resveratrol*

MKN-45-Zellen erhielten die angegebenen Konzentrationen DADS ohne weitere Zusätze (Kontrolle) oder zusammen mit den angegebenen Konzentrationen Resveratrol. Nach 3,5 Stunden wurden die Prozentsätze apoptotischer Zellen in Doppelansätzen bestimmt. Das Ergebnis zeigt, dass der NO Dioxygenasehemmer DADS mit dem NOX1-Stimulator Resveratrol einen konzentrationsabhängigen beachtlichen Synergieeffekt erzeugt.

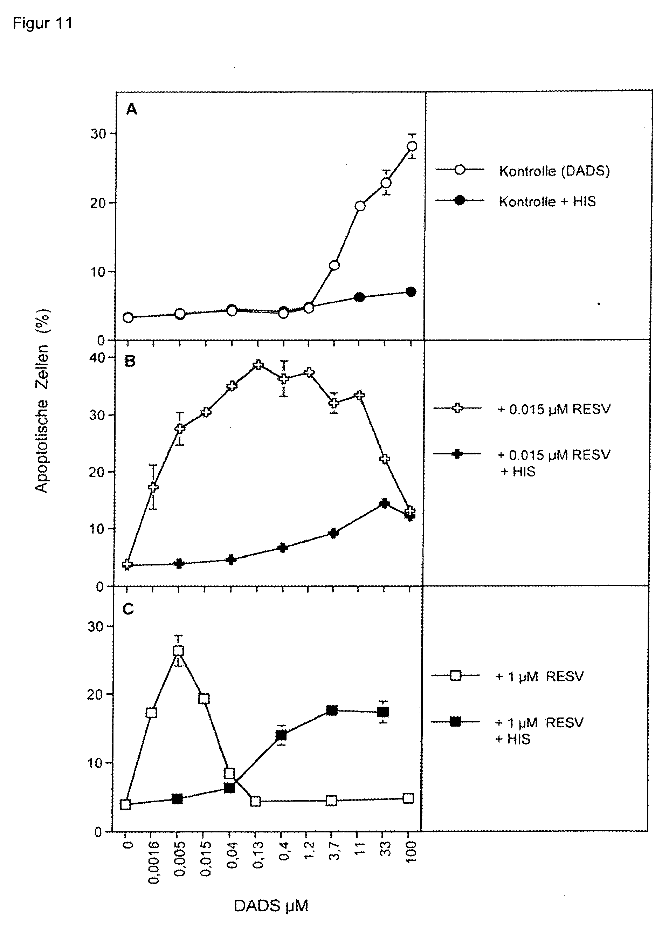

**Figur 11****:** *Der Synergieeffekt zwischen DADS und Resveratrol wird durch Singulettsauerstoff vermittelt.*

Die Wirkung von DADS allein (A) oder der Synergieeffekt zwischen DADS und Resveratrol wird aufgehoben, wenn der Singulettsauerstofffänger Histidin vor DADS zugegeben wurde. Inkubationsbedingungen wie in Figur 10.

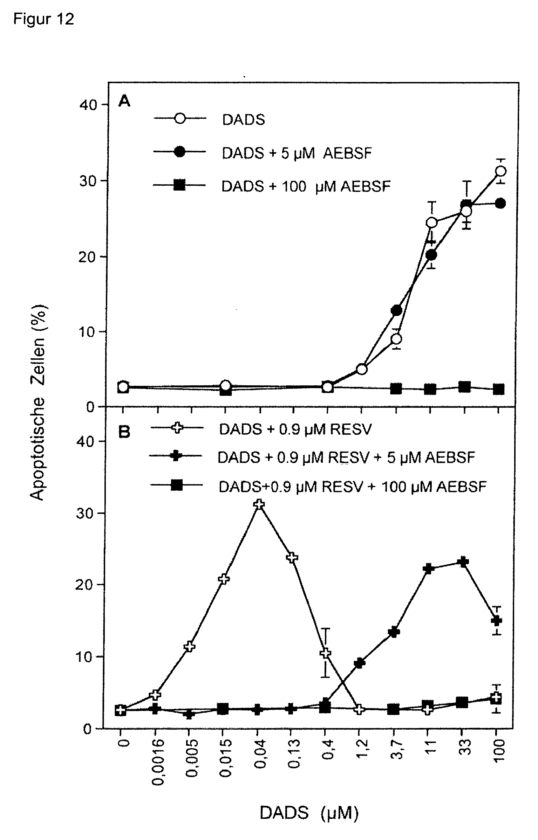

**Figur 12****:** *Der Synergieffekt zwischen DADS und Resveratrol wird aufgehoben, wenn NOX1-Aktivität durch 5 µM AEBSF geringfügig gehemmt wird.*

Inkubationsbedingungen wie in Figur 11. Zusätzlich wurden 5 µM AEBSF oder 100 µM AEBSF zugegeben. 5 µM AEBSF führen zu einer Hemmung um nur 20 % von NOX1, während 100 µM mehr als 90 % hemmen, wie parallel durchgeführte Kontrollmessungen ergeben haben. Die geringfügige Hemmung durch 5 µM AEBSF reicht bereits aus, den Synergieeffekt aufzuheben und die Apoptoseinduktion auf das Niveau der alleinige Gabe von DADS zu drücken. Dies zeigt, dass tatsächlich die NOX-stimulierende Wirkung von Resveratrol für den Synergieeffekt notwendig war.

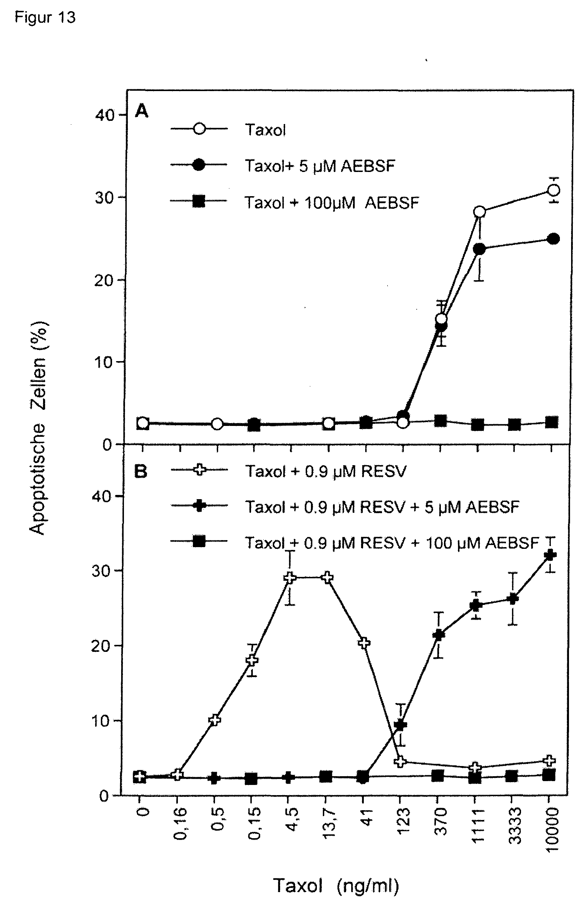

**Figur 13****:** *Der Synergieffekt zwischen Taxol und Resveratrol wird aufgehoben, wenn NOX1-Aktivität durch 5 µM AEBSF geringfügig gehemmt wird.*

Das Experiment wurde analog dem in Figur 12 beschriebenen durchgeführt, mit dem Unterschied, dass Taxol statt DADS verwendet wurde. Das Ergebnis zeigt, dass die NOX-Stimulation durch Resveratrol für dessen Synergieeffekt mit Taxol verantwortlich ist.

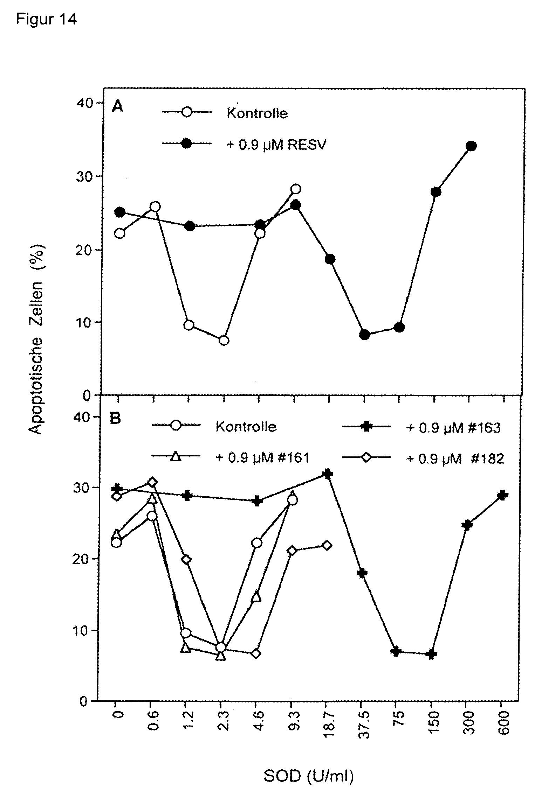

**Figur 14****:** *Steigerung der Superoxidanionenproduktion durch Resveratrol und ein bestimmtes Resveratrol-Derivat*

MKN-45-Zellen unter Standardbedingungen erhielten zunächst keinen Zusatz (Kontrolle) oder 0.9 µM Resveratrol oder der Resveratrolderivate HT 161, HT 163, HT 182, die durch unterschiedliche Blockierung der drei vorhandenen OH-Gruppen gekennzeichnet sind. Dabei waren beim Derivat HT 161 alle drei OH-Gruppen durch TBS (*tert-*Butyldimethylsilyl) blockiert, beim Derivat HT 163 die beiden OH-Gruppen in den Positionen 3'und 5'blockiert und beim Derivat HT 182 selektiv nur die OH-Gruppe in der Position 4'blockiert.

Die Struktur der Vergleichsverbindungen ist nachfolgend wiedergegeben:

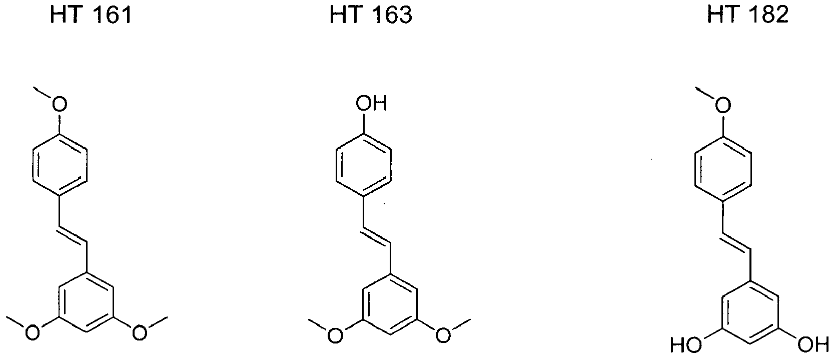

Danach erfolgte die Zugabe von 100 mM 3-AT (Katalasehemmstoff) und der angegebenen Konzentrationen von Cu/ZnSOD. Nach 5,5 Stunden erfolgte die Bestimmung der Prozentsätze apoptotischer Zellen in Doppelansätzen.

Da 3-AT Superoxidanionen-abhängiges interzelluläres Apoptose-auslösendes Signaling reaktiviert, wird der Prozess durch Cu/Zn-SOD blockiert, wobei sich eine typische Glockenkurve ausbildet. Der biochemische Grund dafür wird in Bauer et al., Carcinogenesis 35: 1582-1591, 2014 dargestellt. Bei Erhöhung der Superoxidanionenkonzentration durch Resveratrol ergibt sich eine Rechtsverschiebung der Kurve. Das Resveratrolderivat (163), das eine freie OH-Gruppe in der Position 4 und zwei blockierte weitere OH-Gruppen aufweist ist ebenso wirksam wie Resveratrol selbst, während die Derivate (161) (drei blockierte OH-Gruppen) und (182) (nur OH in Position 4 blockiert) keine stimulierende Wirkung auf die Superoxidanionensynthese zeigen.

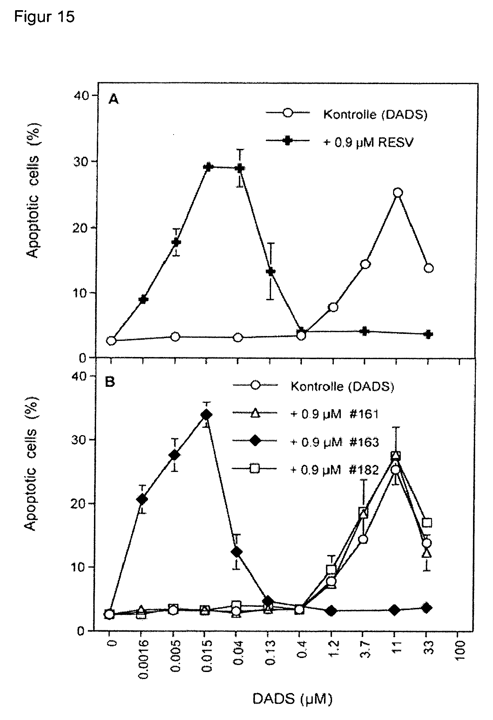

**Figur 15****:** *Korrelation zwischen der stimulierenden Wirkung der Superoxidanionenproduktion und der Induktion eines Synergieeffektes mit DADS*

Die Abbildung zeigt, dass Resveratrol und sein Derivat (163), die sich durch Stimulation der Superoxidanionenproduktion auszeichnen, auch zur Ausprägung eines Synergieeffektes mit DADS befähigt sind, während die auf die Superoxidanionenproduktion unwirksamen Derivate auch nicht zum Synergieeffekt befähigt sind. (Inkubationszeit 5,5 Stunden).

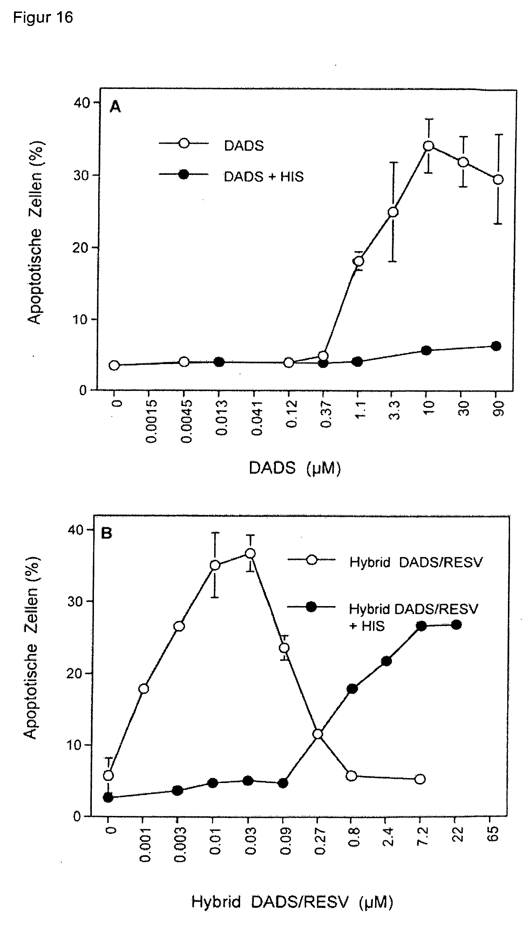

**Figur 16****:** *Das Hybridmolekül aus Diallyldisulfid und Resveratrol ist 1000-fach aktiver als Diallyldisulfid.*

In Figur 16A oben ist die Kontrolle (ohne Hybridmolekül) und unten B mit Hybridmolekül gezeigt.

Das Hybridmolekül wurde aus einem ersten Aromaten, der literaturbekannt ist, und einem zweiten Aromaten, der nicht literaturbekannt ist, in vier Stufen hergestellt, wie in den Beispielen beschrieben. Nach erfolgreicher Synthese des Hybridmoleküls erfolgte die Testung auf biologische Wirksamkeit der neuen Substanz gemäß der DE 10 2005 027 796.

MKN-45 Zellen wurden mit den angegebenen Konzentrationen DADS (Kontrolle) oder des Hybridmoleküls aus DADS und Resveratrol, in Abwesenheit oder Anwesenheit von Histidin für 3,5 Stunden inkubiert, bevor die Prozentsätze apoptotischer Zellen in Doppelansätzen bestimmt wurden. Das Ergebnis zeigt, dass das Hybrid etwa 1000-fach wirksamer ist als DADS und, dass beide Stoffe einen Singulettsauerstoff-abhängigen Prozess anstoßen.

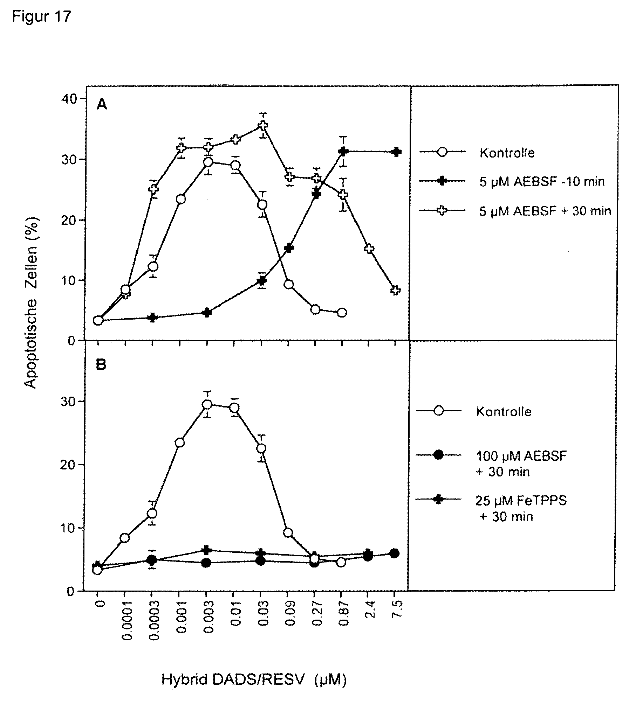

**Figur 17****:** *Die Wirkung des Hybridmoleküls aus DADS und Resveratrol wird durch Superoxidanionen und Peroxynitrit vermittelt.*
**A.** MKN-45 Zellen erhielten keinen Inhibitor (Kontrolle) oder 5 µM) AEBSF 10 Minuten vor oder 30 Minuten nach Zugabe der angegebenen Konzentrationen des Hybridmoleküls. Nach 3,5 Stunden wurden die Prozentsätze apoptotischer Zellen bestimmt. Das Ergebnis zeigt, dass die frühe Zugabe der geringen Konzentration AEBSF den Synergieeffekt aufhebt und den Prozess auf das Niveau der Wirkung von DADS allein zurückfährt, während die Gabe von AEBSF nur 30 Minuten nach DADS zu keiner hemmenden Wirkung führt.
**B.** Die Zugabe von 100 µM AEBSF oder 25 µM FeTPPS 30 Minuten nach Zugabe des Hybridmoleküls hemmt die Apoptoseinduktion vollständig, was zeigt, dass das Signaling, das durch den frühen Effekt des Hybridmoleküls aktiviert wird (Katalasehemmung) über den Peroxynitritweg abläuft.

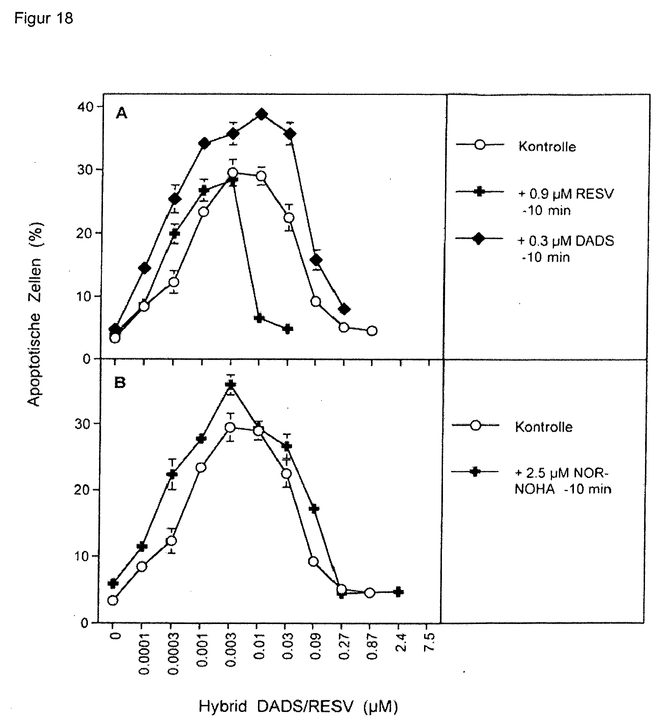

**Figur 18****:** *Die Wirkung des besonders bevorzugten Hybridmoleküls ist nicht weiter steigerbar.*

Die Zugabe von 0,9 µM Resveratrol, 0.3 µM DADS oder 2,5 µM des Arginasehemmers NOR-NOHA können die Wirkung des Hybridmoleküls auf MKN-45 Zellen (Inkubation 3,5 Stunden, Doppelansätze) nicht steigern. Das zeigt, dass die Gestaltung der Struktur des Hybridmoleküls optimal für die beabsichtigte Wirkung ist.

Die für das Verständnis der vorliegenden Erfindung relevanten biochemischen Grundlagen wurden anhand der Figuren näher erläutert. Die folgenden Beispiele erläutern weiterhin die vorliegende Erfindung. Bei den Experimenten wurden folgende Methoden verwendet:

Die Daten basieren allesamt auf der quantitativen Bestimmung der Apoptose mittels der klassischen morphologischen Kriterien Kernkondensation, Kernfragmentation und Membrane Blebbing nach definierten Inkubationszeiten bei 37°C. Parallel wurden diese Kriterien mithilfe der TUNEL-Reaktion gesichert, wodurch die für die Apoptose charakteristischen DNA-Strangbrüche nachgewiesen wurden, und die Abhängigkeit der Apoptoseinduktion von Caspase-3 und Caspase-9 überprüft, wodurch sichergestellt war, dass die Apoptose über den mitochondrialen Weg abläuft.

Die Ansätze erfolgten bei einer Zelldichte von 125 000 Zellen/ml in 96-Loch-Platten mit einem Reaktionsvolumen von 100 µl. Alle Ansätze wurden als Doppelansätze geführt und pro Messpunkt wurden mindestens 200 Zellen morphologisch bewertet. Eine detaillierte Aufstellung der verwendeten Medien, Inhibitoren, Zellen ist in den Arbeiten Scheit and Bauer, 2015; Bauer und Zarkovic, 2014; Bauer et al., 2014; Bauer, 2015 beschrieben.

Der Knockdown durch siRNA wurde in der Arbeit Bauer und Zarkovic, 2014 beschrieben. Dort finden sich auch alle Angaben zu den verwendeten siRNAS.

Die Messung der extrazellulären Superoxidanionenkonzentration von Tumorzellen sowie die Bestrahlungstechnik mit Niedrigdosis-Gammastrahlung wird in der Arbeit Temme and Bauer, 2013 beschrieben. Die Hemmung der NOD wird bei Scheit and Bauer, 2015, beschrieben. Viele Versuchsergebnisse wurden in den Figuren dargestellt.

### Beispiel 1: Synthese des bevorzugten Wirkstoffs

Das Hybrid-Molekül aus Resveratrol und Diallyldisulfid **14** wurde wie in Figur 4 gezeigt synthetisiert. Das Resveratrol-Gerüst (zwei verbundene aromatische Sechsringe) war dabei erstmals in der Zwischenstufe **11** vollständig vorhanden (Figur 4). Diese wurde aus dem Phosphonat **6** und dem Aldehyd **10** in einer Horner-Wadsworth-Emmons-Reaktion aufgebaut(die Synthesen von 6 und 10 sind in den nachfolgenden Absätzen beschrieben).

In einer doppelten nukleophilen Substitution wurden die Chloridfunktionen in **11** durch Thioacetatgruppen ersetzt. Das führte zum Bisthioacetat **12.** Dieses wurde direkt, also ohne zum Bisthiolat zu hydrolysieren, mit Diallyldisulfid und Triethylamin umgesetzt. Das führte zu dem tert-Butyldimethylsilyl-geschützten (TBS-geschützten) unmittelbaren Vorläufer **13** des Hybrid-Moleküls **14.** Nach Entschützen von **13** mit Tetrabutylammoniumfluorid wurde das Hybrid-Molekül **14** (Figur 5) erhalten.

Der Syntheseweg ist in Figur 4 dargestellt und wird nachfolgend wiedergegeben, wobei auch die jeweiligen Reaktionsbedingungen für die einzelnen Schritte a)-l) angegeben sind.

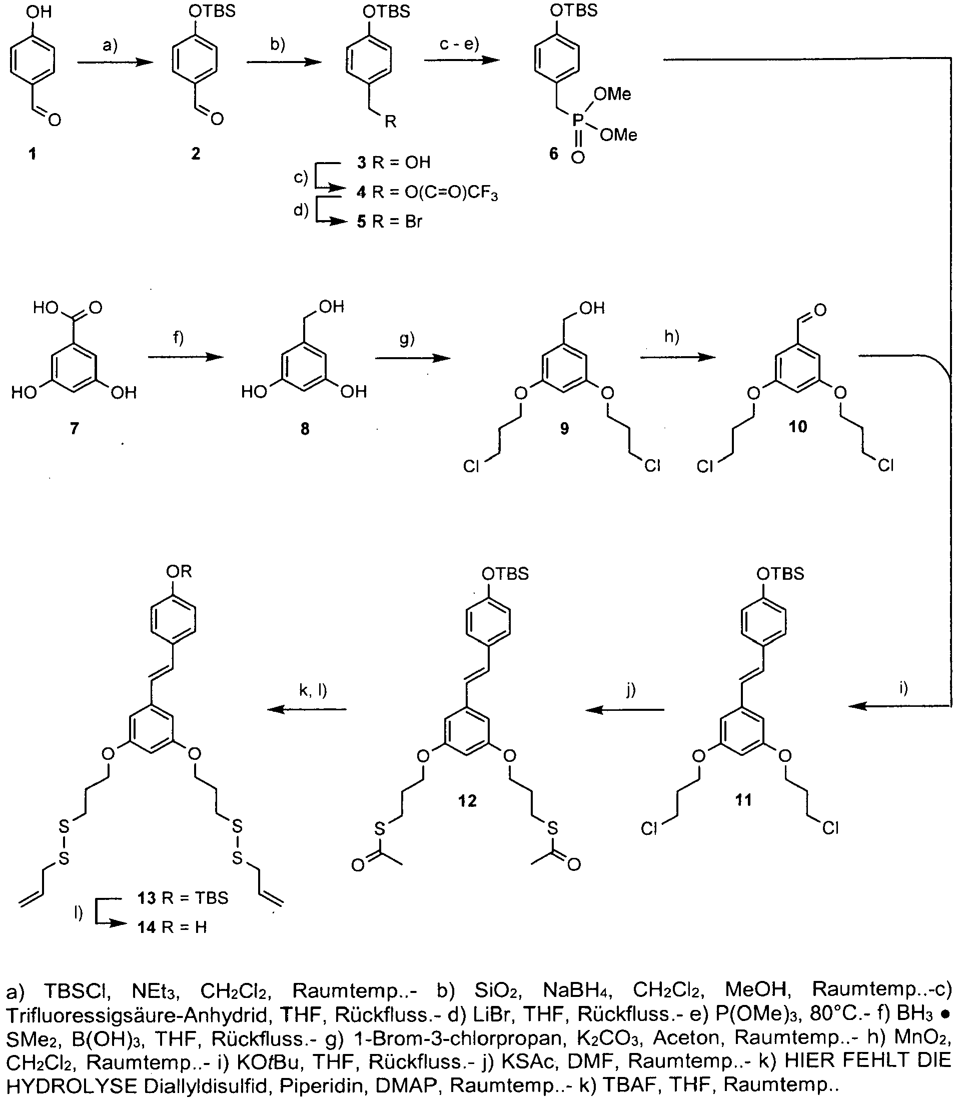

### Beispiel 2: Zwischenprodukte

Das zum Aufbau der Resveratrol-Struktur erforderliche Phosphonat **6** wurde in 5 Stufen aus 4-Hydroxybenzaldehyd **(1)** synthetisiert. Als erstes wurde die phenolische Hydroxygruppe in **1** mit TBS-Chlorid geschützt. Eine anschließende Reduktion des Aldehyds **2** mit NaBH₄ ergab den Benzylalkohol **3.** Er wurde mit Trifluoressigsäure-Anhydrid in das Trifluoracetat **4** überführt. In der folgenden nukleophilen Substitution mit Lithiumbromid wurde das Benzylbromid **5** erhalten. Mit Trimethylphosphit lieferte es in einer Arbuzov-Reaktion das Phosphonat **6.**

Der zum Aufbau der Resveratrol-Struktur erforderliche Aldehyd **10** wurde in 3 Stufen aus 3,5-Dihydroxybenzoesäure **(7)** synthetisiert. Zuerst wurde diese Benzoesäure mit Boran zum Benzylalkohol **8** reduziert. In **8** wurden dann selektiv die beiden phenolischen OH-Gruppen mit 1-Brom-3-chlorpropan verethert. Im daraufhin erhaltenen Bis(chlorpropyl)ether **9** wurde die benzylische OH-Gruppe mit Mangandioxid oxidiert. Das lieferte den Aldehyd **10.**

### Beispiel 3: Aufarbeitung und Reinigung

Die Reinigungsschritte der gesamten Synthese - außer dem allerletzten - wurden durch Flash-Chromatographie an Kieselgel vorgenommen. Die Reinigung des Endprodukts und Hybridmoleküls **14** erfolgte durch präparative HPLC.

Die gewählte erfindungsgemäße Synthesestrategie und das Zielmoleküldesign haben folgende Vorteile:
① Die erfindungsgemäße Synthese ist stark konvergent. Das vergrößert bei einer gegebenen Stufenzahl die Gesamtausbeute.
② Von den 3 OH-Gruppen des Resveratrols muss in bevorzugten Hybrid-Molekülen zumindest die 4'-OH-Gruppe (zur Numerierung vgl. Figur 5) erhalten bleiben. Andernfalls verschwindet die erwünschte Wirkung. Sind demgegenüber die 3-OH-Gruppe und/oder die 5-OH-Gruppe des Resveratrols (zur Numerierung vgl. Figur 5) verethert, wird die erwünschte Wirkung nicht eingebüßt. Insofern ist es zweckmäßig, die spätere 4'-OH-Gruppe aus *einem* Vorläufermolekül mitzubringen (hier: aus dem Phosphonat **6)** und die späteren 3,5-(OH)₂-, 3-OH,5-OR- oder 3,5-(OR)₂-Gruppen aus einem *anderen* Vorläufermolekül (hier: aus dem Aldehyd **10).** Die Horner-Wadsworth-Emmons-Reaktion des Phosphonats **6** (worin die 4'-OH-Gruppe geschützt ist) mit dem Aldehyd **10** (worin die 3- und 5-OH-Gruppen verethert sind) lieferte als Olefinierungsprodukt **11** den Vorläufer eines im obigen Sinn geeigneten Hybridmoleküls (hier **14**): dessen 4'-OTBS-Gruppe ist nämlich am Ende der Synthese in die 4'-OH-Gruppe einer Resveratrol-Struktur überführbar.
③ Die konvergente Natur der erfindungsgemäßen Synthesestrategie eröffnet einen effizienten Zugang zu *weiteren* Hybrid-Molekülen. So kann an den "inneren" Schwefelatomen der beiden Disulfideinheiten anstelle eines Propan-1,3-diylrests eine (ggf. erheblich) längere oder eine um 1 C-Atom verkürzte Kohlenstoffkette eingebracht werden. Das gelingt, indem bei modifizierten Synthesen Analoga des Aldehyds **10** gewonnen werden durch die Verwendung anderer 1,ω-Dialkylantien als 1-Brom-3-chlorpropan. Diese "spacer" dürften natürlich auch andere Strukturen besitzen.
④ Dank unserer Synthesestrategie lassen sich auch die "äußeren" Schwefelatome der beiden Disulfideinheiten bequem anders funktionalisieren als im Hybridmolekül **14,** nämlich im allerletzten Schritt. Das derzeitige Thiylierungsmittel wird dann durch ein anderes Thiylierungsmittel ersetzt.

### Beispiel 4: Experimentelle Bestimmung der Aktivitäten

*Diallyldisulfid und Dipropyldisulfid, nicht aber Diallylsulfid, lösen selektiv in Tumorzellen interzelluläres Signaling und Apoptose aus*

**Figur 6** **A** zeigt, dass die Disulfide Diallyldisulfid (DADS) und Dipropyldisulfid, nicht aber das Monosulfid Diallylsulfid (DAS) in der humanen Magenkarzinomzelllinie MKN-45 Apoptose auslösen, aber ohne Apoptose-induzierende Wirkung auf nichtmaligne, diploide humane Fibroblasten sind.

**Figur 6B** belegt, dass die DADS-vermittelte selektive Apoptoseinduktion in Tumorzellen vollständig gehemmt wird, wenn entweder die NOX1-abhängige Superoxidanionenproduktion mittels des NOX-Inhibitors AEBSF oder die NO-Synthase durch L-NAME gehemmt werden. Die Apoptoseinduktion hängt von der Verfügbarkeit von Singulettsauerstoff ab, da der Singulettsauerstoff-Fänger Histidin diese verhindert. Die Hemmung der Caspase-8 führt ebenfalls zur vollständigen Hemmung der Apoptose, was auf eine Beteiligung des FAS-Rezeptors und der nachgeschalteten Caspase-8 bei der Stimulation von NOX1 hinweist (Bauer, 2012, Scheit and Bauer, 2015). Der HOCI-Fänger Taurin führt zu einer Hemmung der Apoptose im höheren Konzentrationsbereich von DADS, was auf die Wirkung des HOCl-Signalweges hinweist.

### Diallyldisulfid induziert einen Zweistufenmechanismus

**Figur 7** zeigt, dass die Apoptose-auslösende Wirkung von DADS vollständig gehemmt wird, wenn der Singulettsauerstoff-Fänger Histidin (A), der Peroxynitritfänger FeTPPS (B), das Katalasemimetikum EUK-134 (C) oder Caspase-8-Inhibitor (D) vor DADS zur Zellkultur gegeben werden, während deren Gabe 20 min nach DADS-Zugabe die Hemmwirkung fast vollständig aufhebt (Histidin, Caspase-8-Inhibitor) oder auf den niedrigen Konzentrationsbereich von DADS (FeTPPS) oder hohen Konzentrationsbereich von DADS (EUK-134) beschränkt. Im Gegensatz dazu ist die Hemmwirkung des HOCI-Fängers Taurin (E) und des Peroxidasehemmstoffs ABH (F) vom Zeitpunkt der Zugabe unabhängig und beschränkt sich auf den Bereich der höheren DADS-Konzentrationen. DADS induziert also zunächst einen schnellen Singulettsauerstoff-abhängigen Schritt, der von nachfolgendem Apoptose-Signaling durch den NO/Peroxynitritweg (niedrigerer Konzentrationsbereich von DADS) und den HOCl-Weg (höherer Konzentrationsbereich von DADS) gefolgt wird, das mehrere Stunden benötigt. Zur Bildung von Singulettsauerstoff werden H₂O₂ und Peröxynitrit benötigt, die Wirkung des Singulettsauerstoffs ist offensichtlich die Inaktivierung der protektiven membranständigen Katalase, da ohne deren Inaktivierung weder der NO/Peroxynitrit- noch der HOCI-Weg ablaufen könnten.

### Diallyldisulfid induziert die Bildung von Singulettsauerstoff und nachfolgende Inaktivierung der membranständigen Katalase von Tumorzellen

**Figur 8** **A** zeigt, dass die kurzzeitige Behandlung von Tumorzellen mit DADS den gleichen Effekt hat, wie die Zugabe des Katalasehemmstoffs 3-Aminotriazol (3-AT), nämlich die Zellen für die Apoptose-auslösende Wirkung von exogen zugegebenem Peroxynitrit (PON) zu aktivieren. Die Sensitivierung für die Wirkung von PON ermöglicht die selektive Testung der Inaktivierung der membranständigen Katalase von Tumorzellen (Heinzelmann and Bauer, 2010; Böhm et al., 2015). Im Gegensatz zu DADS führt DAS zu keiner Inaktivierung der Katalase **(****Figur 8 F)****.** Die Inaktivierung der Katalase mittels DADS bleibt aus, wenn bei Zugabe von DADS entweder der Singulettsauerstoff-Fänger Histidin **(****Figure 8 B)****,** der NOX1-Inhibitor AEBSF oder das H₂O₂-zerstörende Katalasemimetikum EUK-134 **(****Figur 8 C)****,** der Peroxynitritfänger FeTPPS **(****Figur 8 D)** oder Caspase-8-Inhibitor **(****Figur 8 E)** im Testansatz vorliegen.

Die Zugabe von Histidin oder Caspase-8-Inhibitor nach der Inkubation mit DADS aber unmittelbar vor Applikation von Peroxynitrit führt zu keiner Hemmung der Katalaseinaktivierung. Figur 8 zeigt insgesamt, dass DADS zur Bildung von Singulettsauerstoff aus Peroxynitrit und H₂O₂ führt und dass Caspase-8 dabei eine modulierende Rolle spielt.

### Diallyldisulfid hemmt NO Dioxygenase

**Figur 9** zeigt, dass DADS wirkungsvoll NOD hemmen kann. Eine gleichartige Wirkung lässt sich auch für die Flavonoide Malvidin und Quercitin, sowie für die Tumortherapeutika Taxol und Epothilon B nachweisen. Die Hemmung der NOD wurde von uns vor kurzem als Schlüsselereignis bei der Singulettsauerstoff-abhängigen Katalaseinaktivierung von Tumorzellen nach Gabe des Anthocyanidins Cyanidin nachgewiesen und der zugrunde liegende Mechanismus wurde geklärt (Scheit und Bauer, 2015; Bauer, 2015). Die Daten zeigen, dass DADS, Anthocyanidine, Taxol und Epothilon B die gleiche Zielstruktur, nämlich NO Dioxygenase (NOD) hemmen und dadurch eine analoge Wirkung auslösen, die sich auf Singulettsauerstoffbildung, Katalaseinaktivierung und interzellulärem ROS-Signaling begründet. In Einklang mit dieser Schlussfolgerung ist der Befund, dass diese Wirkstoffe nur Apoptose auslösen können, wenn die Zielzellen membranständige NOX1 aktiviert haben (Daten nicht gezeigt).

### Synergieeffekt zwischen dem NOX Stimulator Resveratrol und dem NOD-Hemmer DADS und Taxol

**Figur 10** belegt, dass DADS und Resveratrol bei der Apoptoseinduktion in Tumorzellen synergistisch zusammenwirken. Der Synergieeffekt ist von den Konzentrationen beider Stoffe abhängig.

Der Synergieeffekt zwischen Resveratrol und DADS ist ebenso, wie die Wirkung von DADS von der Bildung von Singulettsauerstoff abhängig, da er durch Histidin blockierbar ist **(****Figur 11****).**

Der Synergieeffekt zwischen DADS und Resveratrol ist tatsächlich von der Steigerung der NOX-Aktivität durch Resveratrol abhängig, da er durch 5 µM AEBSF (das zu einer 20%igen Reduktion der NOX-Aktivität führt, wie in parallelen Messungen bestimmt wurde) aufgehoben und auf die Wirkung von DADS allein zurückgeführt wird **(****Figur 12****).** Die Wirkung von DADS allein wird durch 5 µM AEBSF nicht inhibiert, während die Zugabe von 100 µM AEBSF (mehr als 90 %ige Hemmung von NOX) erwartungsgemäß zur Hemmung der Apoptose führt.

Das Zusammenspiel von Taxol und Resveratrol führt ebenfalls zu einem Synergieeffekt, der unter anderem durch die NOX-stimulierende Wirkung von Resveratrol bestimmt wird, da er sich durch geringfügige Hemmung der Superoxidanionproduktion durch 5 µM AEBSF aufheben lässt (Figur 13)

### Nachweis der NOX stimulierenden Wirkung von Resveratrol und bestimmter Resveratrolderivate

**Figur 14** zeigt, dass Resveratrol zur Steigerung von NOX1 von Magenkarzinomzellen führt, was sich als Verschiebung der Hemmkurve durch Superoxiddismutase ausdrückt (Temme und Bauer, 2013). Das Resveratrolderivat (163) (das sich durch eine unblockierte OH-Gruppe in der Position 4 auszeichnet [siehe dazu auch Figur 5]) führt im gleichen Ausmaß wie nichtmodifiziertes Resveratrol zur Steigerung der NOX-Aktivität, während Derivate mit blockiertem OH in der Position 4 (161): alle OH-Gruppen blockiert; (182): nur OH-Gruppe in der Position 4 blockiert) ihre Wirkung verloren haben.

**Figur 15** zeigt, dass sich die NOX-stimulierende Wirkung von Resveratrol und seines Derivats mit nicht blockierter OH-Gruppe in Position 4 auch in der Ausprägung des Synergieeffektes mit DADS widerspiegelt, was beweist, dass genau diese Funktion für den Synergieeffekt benötigt wird.

*Dieser überraschende Effekt war eine der zentralen Grundlagen für die Synthese des Hybridmoleküls. Nicht vorhergesehen werden konnte allerdings, ob* es *möglich ist, wesentliche Gruppen eines aktiven Agens mit wesentlichen Gruppen eines anderen Agens so kovalent in einem neuen Molekül zu verbinden, dass die Wirksamkeiten erhalten bleiben, weil die sterische Konformation in den Hybridmolekülen verändert wird und die Folgen unvorhersehbar sind.*

### Beispiel 5: Experimentelle Ableitung notwendiger Strukturmerkmale des vorgestellten Hybridmoleküls

Die bisher vorgestellten Daten zeigen, dass die Disulfidgruppe im DADS-Anteil eines Hybridmoleküls essentiell ist, da die Wirkung bei Vorliegen eines Monosulfids vollständig verloren geht.

Im Gegensatz dazu sind die Allylgruppen verzichtbar und können z. B. durch Propylgruppen ersetzt werden, was belegt, dass die Doppelbindung der Seitengruppen keine biochemische Bedeutung in diesem Kontext hat. Dies gilt auch für die Länge der Seitengruppen, wie sich aus der Struktur des synthetisierten Hybridmoleküls ableiten lässt (Figur 5). Diese Struktur zeigt auch, dass die beiden Seitengruppen verschieden voneinander sein dürfen, ohne die Funktion des Gesamtmoleküls zu gefährden.

Die erfindungsgemäßen Daten zeigen auch, dass die OH-Gruppen in Position 3 und 5 von Resveratrol für eine Koppelung verwendet werden dürfen, ohne dass dadurch die NOX-stimulierende Funktion des Resveratrolanteils beeinträchtigt würde. Dies gilt nicht für die OH-Gruppe in Position 4, deren Blockierung zu einem vollständigen Ausfall der gewünschten Funktion führt.

### Beispiel 6: Charakterisierung der biologischen Eigenschaften des neuen Hybridmoleküls aus Struktur- und Wirkungselementen von Resveratrol und Diallyldisulfid

Das Hybridmolekül aus Struktur- und Wirkungselementen von Resveratrol und Diallyldisulfid bündelt in sich die Synergiewirkung der beiden Komponenten.

Um zu klären, ob das Hybridmolekül aus Struktur- und Wirkungselementen von Resveratrol und Diallyldisulfid in der Lage ist, Apoptose in Tumorzellen zu induzieren, und dabei zu prüfen, ob sich durch die Kombination der Einzelfunktionen in einem Molekül der Synergieeffekt bündeln ließ, wurden steigende Konzentrationen des Hybridmoleküls zu Magenkarzinomzellen der Linie MKN-45 gegeben. Die als Parallelansätze geführten Ansätze wurden sowohl ohne als auch in Anwesenheit von 2 mM des Singulettsauerstofffängers Histidin inkubiert, um zu prüfen, ob der durch das Hybridmolekül ausgelöste Effekt durch Singulettsauerstoff vermittelt wird. Zum Vergleich wurde das gleiche Experiment mit steigenden Konzentrationen DADS durchgeführt, ebenfalls mit und ohne Histidinzugabe. Nach 3,5 Stunden Inkubation wurde der Prozentsatz apoptotischer Zellen bestimmt.

**Figur 16** zeigt, dass das Hybridmolekül aus Resveratrol und Diallyldisulfid bereits bei einer Konzentration von 10 nM den maximalen Apoptose-auslösenden Effekt erzielte, während bei alleiniger Gabe von DADS 10 µM des Wirkstoffs notwendig waren. Bei einer Konzentration von 1 nM des Hybridmoleküls wurden ca. 40 Prozent des Maximalwertes der Apoptoseinduktion erreicht. Für den gleichen Effekt waren 1,1 µM DADS bei alleiniger Gabe notwendig. Diese Verschiebung der Konzentrations-Wirkungskurven belegt, dass das Hybridmolekül aus Diallyldisulfid und Resveratrol etwa 1000-fach wirksamer ist als Diallyldisulfid allein, was sich nur dadurch erklären lässt, dass das Hybridmolekül die Fähigkeit, den Synergieeffekt auszulösen in sich bündelt. Es wäre möglicherweise aus sterischen Gründen naiv, anzunehmen, dass einzelne Hybridmoleküle mit beiden Zielstrukturen gleichzeitig interagieren. Vielmehr darf angenommen werden, dass Hybridmoleküle entweder mit NOD oder mit NOX interagieren und dadurch ein analoger Effekt ausgelöst wird, wie bei gleichzeitiger Gabe der Einzelsubstanzen. Allerdings sichert die Anwendung eines Hybridmoleküls, dass in der angestrebten therapeutischen Situation in vivo beide Komponenten am gleichen Ort vorhanden sind. Der Befund lässt auch den Schluss zu, dass die Affinität der beiden Zielstrukturen für das Hybridmolekül so ausgewogen ist, dass es zu keiner Beeinträchtigung einer der beiden Strukturen kommt.

Sowohl die Wirkung von Diallyldisulfid alleine als auch die des Hybridmoleküls wird durch Singulettsauerstoff vermittelt, wie die Hemmbarkeit durch den Singulettsauerstoff-Fänger Histidin belegt. Dies legt nahe, dass das Hybridmolekül den gleichen Wirkmechanismus nutzt, der auch für DADS erarbeitet wurde. Dieser ist durch die Hemmung der NOD, Anstieg der lokalen NO-Konzentration, transiente Hemmung der Katalase, Bildung von Singulettsauerstoff aus H₂O₂ und Peroxynitrit, Inaktivierung der membranständigen Katalase durch Singulettsauerstoff und nachfolgendes interzelluläres Apoptose-auslösendes Signaling charakterisiert. Der Anstieg der Kurve in Gegenwart von Histidin bei sehr hohen Konzentrationen an Hybridmolekül in Figur 16B ist wohl dem Verbrauch des Inhibitors in diesem Konzentrationsbereich geschuldet.

Um zu klären, ob der durch das Hybridmolekül gebündelte Synergieeffekt tatsächlich durch die NOX-stimulierende Wirkung des von Resveratrol abgeleiteten Anteils mitgetragen wird, wurde die Wirkung des Hybridmoleküls in Gegenwart von 5 µM AEBSF untersucht. Für diese Hemmstoffkonzentration wurde in Parallelexperimenten ermittelt, dass sie eine Hemmung der extrazellulären Superoxidanionenproduktion um ca. 20 % zur Folge hat. **Figur 17** zeigt auch, dass die Anwesenheit von 5 µM AEBSF vor Zugabe des Hybridmoleküls die Konzentrations-Wirkungskurve sehr deutlich nach rechts verschiebt. Dies belegt, dass NOX-abhängige Superoxidanionenproduktion für die außerordentlich hohe Effizienz des Hybridmoleküls mitverantwortlich ist und, dass bereits eine relativ geringe Minderung der offensichtlich limitierenden Superoxidanionkonzentration zum Abbruch des Synergieeffektes beiträgt. (Dieser Befund ist analog zu dem in Figur 12 gezeigten Effekt auf die Synergiewirkung der einzeln gegebenen Wirkstoffe). Im Gegensatz zur Gabe von 5 µM AEBSF vor Zugabe des Hybridmoleküls führt die Gabe von 5 µM AEBSF 30 Minuten nach Zugabe des Hybridmoleküls zu keiner Rechtsverschiebung der Konzentrations-Wirkungskurve, sondern zu deren Verbreiterung **(****Figur 17 A)****.** Zu diesem Zeitpunkt ist der in Bezug auf die Superoxidanionenkonzentration sehr kritische Vorgang bereits abgeschlossen. Die Verbreiterung der Optimumskurve ist dadurch erklärbar, dass eine supraoptimale Katalaseinaktivierung, die zu graduellen Abbruch des HOCI-Signalwegs führt verhindert wurde. Die Zugabe von 100 µM AEBSF 30 Minuten nach Zugabe des Hybridmoleküls führt zu einer vollständigen Hemmung der Apoptoseinduktion **(****Figur 17 B)****.** Diese Konzentration des Inhibitors hemmt die Superoxidanionensynthese um mehr als 95 %. Dieses Ergebnis zeigt daher, dass die Wirkung des Hybridmoleküls vollständig auf interzellulärem ROS-abhängigen Signaling begründet ist, bei dem Superoxidanionen eine Schlüsselrolle spielen. Bei diesem Apoptose-auslösenden Signaling handelt es sich ausschließlich um den NO/Peroxynitritweg, wie die vollständige Hemmbarkeit durch den Peroxynitritfänger FeTPPS belegt **(****Figur 17 B)****.** Da der Peroxynitritweg in Tumorzellen durch membranständige Katalase wirkungsvoll unterdrückt wird, darf aus diesem Ergebnis auch geschlossen werden, dass das Hybridmolekül in einem ersten Schritt die Inaktivierung der membranständigen Katalase induziert haben muss. Die Abhängigkeit der Wirkung des Hybridmoleküls von Singulettsauerstoff **(****Figur 16****)** weist dabei auf den oben skizzierten zugrundeliegenden Mechanismus, in Analogie zu dem kürzlich beschriebenen Weg (Scheit K et al., Carcinogenesis 36: 400-411 (2015); Bauer G et al., Redox Biol 6: 353-371 (2015)).

### Beispiel 7: Die Wirksamkeit des Hybridmoleküls aus Resveratrol und Diallyldisulfid ist durch zusätzliche Gabe von Resveratrol, Diallyldisulfid oder eines Arginasehemmstoffes nicht weiter steigerbar

Es war zu klären, ob der gebündelte Synergieeffekt durch das Hybridmolekül bereits optimal war, oder ob durch Ergänzung definierter Einzelkomponenten eine weitere Steigerung der Wirkung zu erzielen sein könnte. Zur Klärung dieser Frage wurde die Apoptose-auslösende Wirkung des Hybridmoleküls in Abwesenheit und in Gegenwart einer substanziellen Konzentration von Resveratrol, DADS oder des Arginasehemmstoffs NOR-NOHA gemessen. Figur 18 zeigt, dass die Wirksamkeit des Hybridmoleküls auch dann nicht weiter gesteigert werden kann, wenn entweder ein 300-facher Überschuss an Resveratrol oder ein ca. 100-facher Überschuss an DADS zugegeben werden **(****Figur 18 A****).** Ebenso führt die Zugabe des Arginasehemmstoffs NOR-NOHA zu keiner weiteren Steigerung der Wirkung des Hybridmoleküls **(****Figur 18 B)****.** Für NOR-NOHA wurde von uns bereits gezeigt, dass es einen synergistischen Effekt mit NOD-Hemmstoffen bewirken kann (Bauer G et al., Redox Biol 6: 353-371 (2015)). *Diese Befunde belegen, dass der gewählte molekulare Aufbau des Hybridmoleküls optimal für dessen sehr effiziente Apoptose-induzierende Wirkung auf Tumorzellen ist und in unerwarteter Weise mit gängigen Verfahren nicht weiter steigerbar erscheint.*

### Beispiel 8: Vergleich der Wirksamkeiten von Diallyldisulfid und Taxol mit der des Hybridmoleküls aus Resveratrol und Diallyldisulfid, und Bewertung des Gesamtergebnisses

Aus den in dieser Erfindungsmeldung gezeigten Daten lässt sich ein direkter Vergleich der biologischen Wirkung einzelner Wirkstoffe oder deren Kombinationen mit der des Hybridmoleküls aus Schlüsselstrukturen von Resveratrol und Diallyldisulfid mit beeindruckendem Gesamtergebnis erstellen:

| Tabelle 2 | |
|---|---|
| **Wirkstoff** | **Konzentration für optimale Wirkung (nM)** |
| DADS | 10 000 - 44 000 |
| Taxol | 900 - 3800 |
| DADS (+ Resveratrol) | 15 |
| Taxol (+ Resveratrol) | 48 |
| Hybridmolekül | 3 - 30 |

Tabelle 2 zeigt, dass das etablierte Tumortherapeutikum Taxol mindestens 10-fach wirksamer in Bezug auf Induktion der Apoptose in Tumorzellen ist als der NOD-Hemmstoff Diallyldisulfid, für den zumindest im Tierexperiment eine Anti-Tumorwirkung belegt ist. Durch Nutzung des Synergieeffektes zwischen dem NOX-Stimulator Resveratrol und den NOD-Hemmstoffen Taxol oder Diallyldisulfid (bei Zugabe der Kombination der Einzelstoffe) lässt sich jedoch eine dramatische Steigerung der Wirksamkeiten beider Substanzen erzielen, die nun in der gleichen Größenordnung liegen. Der Synergieeffekt überdeckt also die höhere Wirksamkeit von Taxol gegenüber DADS, wie sie bei der Anwendung der Einzelsubstanzen erkennbar ist.

Das Hybridmolekül aus Schlüsselstrukturen von Resveratrol und Diallyldisulfid ist ebenso wirksam wie die optimale Kombination von einzeln gegebenem Diallyldisulfid plus Resveratrol oder Taxol plus Resveratrol. Damit ist belegt, dass die Bündelung des Synergieeffektes in ein Hybridmolekül gelungen ist.

Aufgrund der gleichartigen Wirkung von Taxol und Diallyldisulfid, die durch diese Erfindungsmeldung belegt ist und der in Tabelle 1 gezeigten Werte kann daher gefolgert werden, dass das Hybridmolekül aus Resveratrol und Diallyldisulfid nicht nur dramatisch wirksamer ist als Diallyldisulfid bei alleiniger Gabe, sondern dass dieses Hybridmolekül sehr deutlich wirksamer sein sollte als Taxol, vorausgesetzt die Befunde in vitro lassen sich auf die Situation in vivo übertragen. Es kann auch vermerkt werden, dass trotz der höheren Wirksamkeit von Taxol im Vergleich zu Diallyldisulfid (bei jeweils alleiniger Gabe) ein denkbares Hybridmolekül aus Resveratrol und Taxol keinen Vorteil gegenüber dem offenbarten Hybridmolekül aus Resveratrol und Diallyldisulfid bringen sollte, da sich dieser sonst bei dem Synergieexperimenten mit kombinierten Einzelsubstanzen hätte zeigen müssen.

Die Synthese eines erfindungsgemäßen Hybridmoleküls bietet zunächst gegenüber der Gewinnung von Taxol oder der Synthese eines hypothetischen Hybridmoleküls zwischen Resveratrol und Taxol einen immensen ökonomischen Vorteil, da auf relativ günstig zu erhaltende Ausgangschemikalien zurückgegriffen werden kann. Für die Herstellung von Taxol ist hingegen immer noch die Gewinnung eines Ausgangsstoffes aus der geschützten Kalifornischen Eibe notwendig, was sowohl eine Limitierung des Materials als auch sehr hohe Kosten mit sich bringt.

Die hohe Wirksamkeit des Hybridmoleküls in vitro lässt auf die Entwicklung eines hochwirksamen Medikaments für die Anwendung in vivo schließen, dessen niedrige Konzentration zu einer Minderung oder Ausschaltung von unerwünschten Nebenwirkungen führen sollte. Derartige Nebenwirkungen sind für die derzeitige Therapie mit Taxol noch von erheblicher Bedeutung.

Ein wesentlicher Vorteil der Bündelung des Synergieeffektes in einem Hybridmolekül gegenüber der Nutzung des Synergieeffektes bei Kombination der Einzelsubstanzen liegt vor allem darin, dass für die Austestung der wichtigen biologischen und therapeutischen Parameter eines Hybridmoleküls keine Schachbrett-artigen Kombinationen der Wirkstoffe eingesetzt werden müssen, sondern dass sich eine einfache lineare Beziehung zwischen der Konzentration des Hybridmoleküls und der betrachteten Wirkung ergeben sollte. Dies führt erkennbar zu einer dramatisch günstigeren Kostensituation bei der Etablierung und Prüfung dieses potenziellen Anti-Tumor-Medikaments.

## Patentansprüche

1. Verbindung der allgemeinen Formel (I) 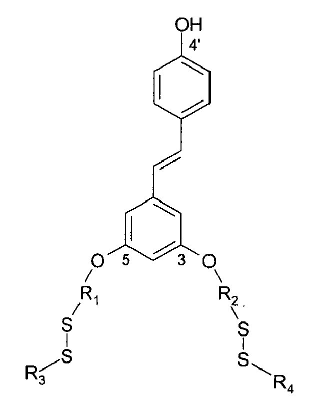 worin die Reste R₁ und R₂ unabhängig voneinander die Bedeutung (CH₂)ₙ haben, wobei n eine ganze Zahl zwischen 1 und 10 ist und R₃ und R₄ unabhängig voneinander ein Alkylrest mit 1 bis 10 C-Atomen, ein Alkenylrest mit 2 bis 10 C-Atomen, ein Allylrest oder ein Cycloalkylrest mit 5 bis 10 C-Atomen ist.

2. Verbindung nach Anspruch 1, wobei die Verbindung die nachfolgende Formel (II) aufweist:

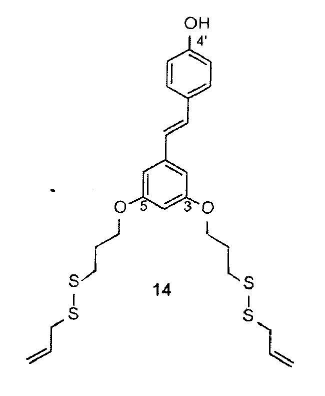

3. Arzneimittel, **dadurch gekennzeichnet, dass** es eine pharmakologisch ausreichende Menge einer Verbindung der Formel (I) oder der Formel (II) oder deren pharmazeutisch annehmbaren Salze beinhaltet.

4. Arzneimittel nach Anspruch 3, **dadurch gekennzeichnet, dass** es zur Behandlung von Tumorerkrankungen geeignet ist.

5. Verwendung einer Verbindung nach Anspruch 1 oder Anspruch 2 zur Behandlung einer Tumorerkrankung.
